# EUROPEAN PATENT SPECIFICATION

(11) **EP 4 299 758 B1**
(45) Date of publication and mention of the grant of the patent: **19.11.2025**
(21) Application number: 22896845.9
(22) Date of filing: 11.08.2022
(51) Int. Cl.: C12Q 1/6816, C12Q 1/6811

(54) **PROBE FOR TARGET ENRICHMENT OF NUCLEIC ACID**
SONDE ZUR GEZIELTEN ANREICHERUNG VON NUKLEINSÄURE
SONDE POUR L'ENRICHISSEMENT CIBLE D'ACIDE NUCLÉIQUE

(30) Priority: 16.05.2022 CN 202210530059
(43) Date of publication of application: 03.01.2024
(73) Proprietor: Nanodigmbio (Nanjing) Biotechnology Co., Ltd, Jiangsu 210000 (CN)
(72) Inventor: WANG, Biao, Jiangsu 210000 (CN); YU, Liping, Jiangsu 210000 (CN); WU, Qiang, Jiangsu 210000 (CN)
(74) Representative: Huang, Liwei
(86) International application number: PCT/CN2022/111610
(87) International publication number: WO 2023/221307

(56) References cited:
- WO-A1-2014/020137
- CN-A- 101 225 432
- CN-A- 104 977 280
- CN-A- 107 034 315
- CN-A- 114 891 859
- US-A1- 2014 323 316
- YANG LINLIN ET AL: "Highly Selective and Sensitive Electrochemiluminescence Biosensor for p53 DNA Sequence Based on Nicking Endonuclease Assisted Target Recycling and Hyperbranched Rolling Circle Amplification", ANALYTICAL CHEMISTRY, vol. 88, no. 10, 27 April 2016 (2016-04-27), US, pages 5097 - 5103, XP093185891, ISSN: 0003-2700, DOI: 10.1021/acs.analchem.5b04521
- YANG XIANG ET AL: "Terahertz spectroscopy for the isothermal detection of bacterial DNA by magnetic bead-based rolling circle amplification", ANALYST, vol. 142, no. 24, 1 January 2017 (2017-01-01), UK, pages 4661 - 4669, XP093186025, ISSN: 0003-2654, DOI: 10.1039/C7AN01438D
- DICKSON ZACHERY W. ET AL: "Probe design for simultaneous, targeted capture of diverse metagenomic targets", CELL REPORTS METHODS, vol. 1, no. 6, 1 October 2021 (2021-10-01), pages 100069, XP093186031, ISSN: 2667-2375, DOI: 10.1016/j.crmeth.2021.100069
- DICKSON ZACHERY ET AL: "Probe design for simultaneous, targeted capture of diverse metagenomic targets", CELL REPORTS METHODS, 25 November 2021 (2021-11-25), XP093186036, Retrieved from the Internet <URL:https://www.sciencedirect.com/science/article/pii/S2667237521001211?via%3Dihub#mmc1>
- WANG HAIXIA ET AL: "Ratiometric Electrochemical Biosensor for the Sensitive Determination of DNA by a Hairpin DNA Probe", ANALYTICAL LETTERS, vol. 54, no. 15, 12 January 2021 (2021-01-12), US, pages 2473 - 2483, XP093186117, ISSN: 0003-2719, DOI: 10.1080/00032719.2020.1871001
- SUNDBERG S O ET AL: "Quasi-digital PCR: Enrichment and quantification of rare DNA variants", BIOMEDICAL MICRODEVICES, SPRINGER US, NEW YORK, vol. 16, no. 4, 1 August 2014 (2014-08-01), pages 639 - 644, XP002784871, ISSN: 1387-2176, DOI: 10.1007/S10544-014-9866-0
- LUO ZHI-MEI, ZHANG YONG-BIAO, CHUN YAN, YUAN-YUAN HAN, RUI H U, LIU JI-QIANG: "Research Advance and Application of Molecular Inversion Probe Technology", BIOTECHNOLOGY BULLETIN, vol. 34, no. 10, 31 December 2018 (2018-12-31), pages 49 - 57, XP093081219, DOI: 10.13560/j.cnki.biotech.bull.1985.2018-0367
- GYDUSH, G. ET AL.: "Massively Parallel Enrichment of Low-Frequency Alleles Enables Duplex Sequencing at Low-Depth Duplex Sequencing", NATURE BIOMEDICAL ENGINEERING, vol. 6, no. 3, 17 September 2022 (2022-09-17), pages 257 - 266, XP037766525, DOI: 10.1038/s41551-022-00855-9

## Description

### Field

The present invention relates to a probe, in particular a set of probes for nucleic acid hybridization capture, method for obtaining a set of probes, and use thereof.

### Background

A nucleic acid sequence is a carrier of life information, while a high-throughput sequencing technology has become one of the core technologies in the biological and medical fields. High-throughput sequencing produces a large amount of data, not all of which are target sequences for research or detection. Although the cost of sequencing has been significantly reduced, due to the high volume of whole genome sequencing data, the cost is still high, and a solution to this problem is to change whole genome sequencing into a targeted enrichment technique. A target region-enriched NGS sequencing technique will ignore information from regions of non-interest in a genome and amplify signals from a target region in the genome, which can save the sequencing cost and the sequencing time.

Targeted enrichment is mainly divided into multiplex PCR amplification and targeted capture based on different enrichment principles. The latter is a probe-based liquid-phase hybrid capture technology, is a mainstream at present, and has the advantages of low probe design difficulty and high probe fault tolerance. The liquid-phase hybrid capture technology is that a biotin-labeled probe specifically binds to a target region in a solution, and target fragments captured by the probe are enriched by streptavidin magnetic beads. During this process, the probe labeled with biotin and liquid phase reaction conditions of hybrid capture have a significant impact on the capture efficiency of this system. For a large target region, the hybrid capture efficiency is higher, for example, a whole exon target region (Panel, also known as a capture region) has an on-target rate of 80% or more; however, for some small target regions (Panels), the on-target rate is relatively low, for example, the on-target rate of a small target region of 10 kb or below may even be lower than 10%.

The selection of a probe sequence length has various considerations: first, the probe length should ensure that in a specific hybridization system, under different sequence base compositions, the hybridization annealing temperature is appropriate, and the binding ability and specificity of the probe with a target sequence are optimal; secondly, it should be ensured that when there is a certain degree of mismatch between sequences of the probe and the target sequence, the hybridization annealing temperature does not decrease significantly; and finally, the longer the probe, the more difficult to synthesize it, and the more difficult to ensure the quality of synthesis. Currently, based on the above considerations, the probe sequence length is generally 40-120 nt, while the mainstream probe length is 120 nt, and is modified (such as biotin), and its modified group can bind to a corresponding affinity medium to complete the "capture" of the target sequence. The forms of the probe include single-stranded DNA, double-stranded DNA, single-stranded RNA, double-stranded RNA, and the like.

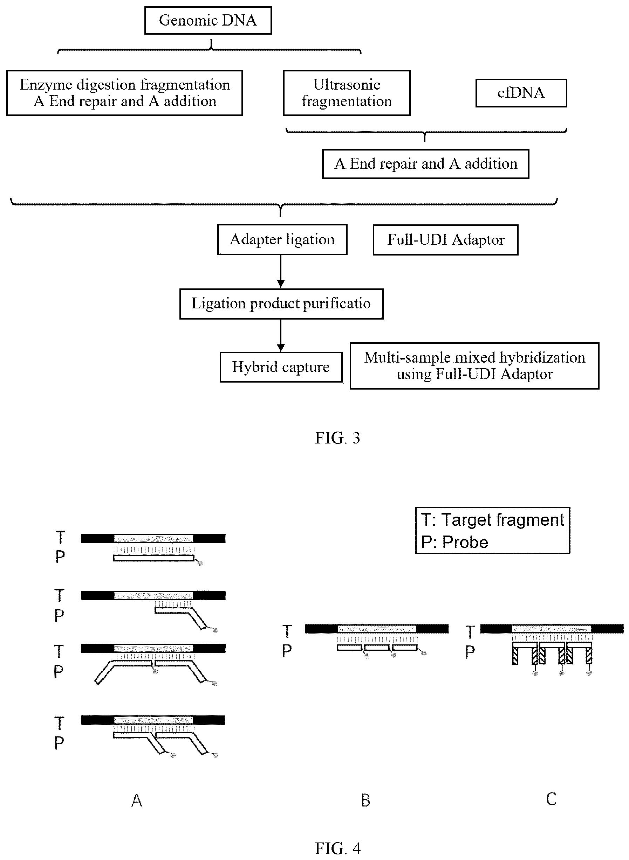

Currently, a second generation sequencing technology is the most widely used high-throughput sequencing technology, with bi-directional 150 bp being a more mainstream sequencing reading mode. The average insert fragment length of a sequencing library is also 100-400bp. The middle part of an excessively long insert fragment cannot be read, and the excessively long fragment also poses a challenge to multiple PCR amplification steps in the sequencing process. In addition, for samples with a short original length, such as FFPE and extracellular free nucleic acid, it is impossible to prepare a library with longer insert fragments. Then, one library molecule typically can only bind to 1-2 probes during hybrid capture, which also means that the probability of probe detachment increases and the recovery rate of the target sequence decreases. For example, a target sequence of 120 bp in length can only bind to one probe completely at most, and even if the target sequence can bind to two probes, the two probes can only be partially bound. In order to increase the binding capacity and probability of probes, the probes may be shortened and the number of the probes may be increased, or an imbricated design strategy may be adopted, i.e., the probes are overlapped with each other so that different target sequence fragments have a higher probability of more complete binding to the probes. However, even probes which are overlapped with each other cannot completely bind to the same target fragment simultaneously (FIG. 4A).

For a sequencing library subjected to PCR amplification, there are multiple copies in each target fragment, and therefore a lower recovery rate can also ensure that most of the original target fragments have captured copies. And the hybrid capture technology typically targets regions of 5 kb or more, while for inherent non-specific capture, compression can be performed by a variety of means, with an on-target rate (a proportion of a target sequence in all captured sequences) being guaranteed to a certain extent. However, current mainstream probes and hybrid capture systems do not provide a satisfactory recovery efficiency and on-target rate for a sequencing library that has short insert fragments, or is not subjected to PCR amplification, and an application requirement with a low proportion of target regions in total regions.

In addition, the liquid-phase hybrid capture process is very time-consuming, taking 2-4 days from a nucleic acid sample to capture library obtaining; meanwhile, hybrid capture involves a large number of reagents, is an extremely cumbersome operation process, and has high technical requirements for operators. A problem in any link of the process will affect the performance of the capture library. These links become critical technical bottlenecks that restrict the development of liquid-phase hybrid capture.

The liquid-phase hybrid capture technology is widely used in cancer tumor mutation gene detection, copy number variation, and methylation status analysis. At present, many products are applied to gene detection and clinical application research in the market. However, with the rise in the popularity of early screening of tumors and MRD, higher requirements are put forward for the liquid-phase hybrid capture technology. For example, for a solid tumor MRD detection technology, primary tumor tissue is first sequenced to identify patient-specific genomic variation maps, and then a target region is designed for personalized ctDNA detection analysis. This requires higher requirements for a hybrid capture system in terms of compatibility with small target regions, ease of operation, degeneracy of experimental processes, and degree of automation.

Therefore, developing a probe with high recovery efficiency and a high on-target rate, as well as a liquid-phase hybrid capture system with high capture efficiency, uniformity, stability, and easy operation, fewer types of reagents, and short time consumption is a solution to solve the problems in the current market.

Some prior arts can be seen in the following documents:
D1: YANG LINLIN ET AL: "Highly Selective and Sensitive Electrochemiluminescence Biosensor for p53 DNA Sequence Based on Nicking Endonuclease Assisted Target Recycling and Hyperbranched Rolling Circle Amplification", ANALYTICAL CHEMISTRY, vol. 88, no. 10, 27 April 2016 (2016-04-27), pages 5097-5103
D2: YANG XIANG ET AL: "Terahertz spectroscopy for the isothermal detection of bacterial DNA by magnetic bead-based rolling circle amplification", ANALYST, vol. 142, no. 24, 1 January 2017 (2017-01-01), pages 4661-4669
D3: DICKSON ZACHERY W.ET AL: "Probe design for simultaneous, targeted capture of diverse metagenomic targets", CELL REPORTS METHODS, vol. 1, no. 6, 1 October 2021 (2021-10-01), page 100069
D4: WANG HAIXIA ET AL: "Ratiometric Electrochemical Biosensor for the Sensitive Determination of DNA by a Hairpin DNA Probe", ANALYTICAL LETTERS, vol. 54, no. 15, 12 January 2021 (2021-01-12), pages 2473-2483.

### Summary

The present invention provides a probe for nucleic acid capture and enrichment, and a design method for a pool of probes that is composed of the probe.

The invention is set out in the appended set of claims.

The present invention provides a design method for a pool of probes for nucleic acid capture and enrichment, including the following steps of:
a) inputting initial sequence information and design parameters, and outputting probe sequence information, wherein the initial sequence information includes (1) total sequence information, which is information of sequences that are possible to be contained before capture in a library; and (2) target sequence information, which is information of sequences to be captured, and sequences that need to be avoided, i.e., low-specificity sequences such as repeated sequences in a comprehensive sequence; and
   the design parameters include an annealing temperature range and a sequence length range for binding a probe to a target sequence, and a length range of a binding sequence between probes;
b) slicing out all subsequences with a length of k, wherein k is 20-80 nt, from forward strand sequences and complementary strand sequences in the total sequence, and counting the number of occurrences of each subsequence;
c) selecting a probe binding sequence in which probes are complementary paired, wherein the probe binding sequence has a length of k, wherein k is 8-30 nt, and has an annealing temperature that is lower than the annealing temperature for binding the probes to the target sequence, and occurs less frequently in the total sequence, and preferably, the number of occurrences of the probe binding sequence in the total sequence is less than 5% of the average;
d) selecting target specific sequences in which the probes bind to a nucleic acid target sequence, wherein an ith target sequence is selected, i having an initial value equal to 1; and the target specific sequences in which the probes bind to the nucleic acid target sequence are then selected, starting from an nth base of the selected target sequence, n having an initial value equal to 1;
e) adding the probe binding sequence to a 5' end of each target specific sequence, and adding a reverse complementary sequence of the probe binding sequence to a 3' end of each target specific sequence; and
f) outputting all probe sequences.

Preferably, if a target specific sequence in which a probe binds to the nucleic acid target sequence does not fall into a sequence interval that needs to be avoided, the target specific sequence is added to the pool of probes, and m1 bases are spaced to try to obtain a next target specific sequence; and if the target specific sequence in which the probe binds to the nucleic acid target sequence falls into the sequence interval that needs to be avoided, the target specific sequence is not added to the pool of probes, and m2 bases are spaced to try again to obtain a target specific sequence;
wherein the number m1 is greater than or equal to the length of the target specific sequence; and the number m2 is less than or equal to a minimum value of a length range of the target specific sequence.

Preferably, selecting the target specific sequences in which the probes bind to the nucleic acid target sequence includes the steps of: when n is less than a length of the ith target sequence, selecting a next target specific sequence; when n is greater than or equal to the length of the ith target sequence, finishing the selecting for the ith target sequence; and after selection of the target specific sequences for the ith target sequence is finished, performing the above selection of target specific sequences on an i+1th target sequence until selection of target specific sequences is completed for all target sequences.

The present invention also provides use of the probe described above for low-frequency mutation detection, chromosome copy number variation analysis, insertion/deletion, microsatellite instability or fusion gene variation in DNA fragments.

The present invention also provides use of the probe described above for targeted metagenomic next-generation sequencing (mNGS) or detection of pathogen epidemiology.

Compared with the prior art, the probes of the present invention have the beneficial effects that the probes of the present invention bind more firmly to a target fragment compared with conventional probes, and the number of probes to which the target fragment can bind can be increased by a shorter target specific binding sequence. The probes of the present invention are more suitable for capture of short fragment libraries; are more suitable for capture of small target regions; are more suitable for capture of PCR-free libraries; and are more conducive to shortening of a hybrid capture process.

### Brief Description of the Drawings

The accompanying drawings forming part of this application are intended to provide a further understanding of the present invention. The illustrative examples of the present invention and explanations thereof are intended to explain the present invention and do not constitute an improper limitation of the present invention. In the accompanying drawings:
FIG. 1 is a structural schematic diagram of probes according to the present invention, wherein each probe is mainly composed of 4 parts: a P-Cap region complementary to target genes, a P-L region at a 3' end, and a P-R region at a 5' end, wherein the 5' end of each probe is labeled with biotin, and the P-L and the P-R have a sequence complementary to each other.
FIG. 2 is a graph comparing a flow of a conventional hybrid capture system and a flow of a hybrid capture system of the present invention.
FIG. 3 is an experimental protocol for different types of samples.
FIG. 4 is a structural schematic diagram of binding a conventional probe (A) of 120 nt, a short probe (B) used in the prior art, and the probe (C) of the present invention to a target fragment, wherein T represents a target fragment of a sample nucleic acid, and P represents the probe.
FIG. 5 is an experimental result of hybrid capture library NGS for the conventional probes of 120 nt, the short probes used in the prior art, and the probes of the present invention.
FIG. 6 shows the capture effect of the conventional probes of 120nt and the probes of the present invention for a PCR-free library.
FIG. 7 shows a concentration test result of the probes according to the present invention.
FIG. 8 shows a hybridization temperature test result of the probes according to the present invention.
FIG. 9 shows a hybridization time test result of the probes according to the present invention.

### Detailed Description of the Embodiments

The present invention will be further described below with reference to the accompanying drawings and specific examples.

All technical and scientific terms used herein have the same meaning as that commonly understood by those skilled in the art to which the present invention belongs. In other cases, certain terms used herein will have their meanings set forth in the specification. Experimental methods in which specific conditions are not indicated in the following examples are within the general knowledge and common general knowledge of those skilled in the art. The examples in this application and the features in the examples can be combined with each other.

The features and advantages of the present invention will be further understood from the following detailed description in conjunction with the accompanying drawings. The examples provided are merely illustrative of the method of the present invention, and are not intended to limit the rest of the contents of the present invention in any way.

The present invention provides a set of probes for nucleic acid capture, wherein the probes are designed separately for a positive sense strand and a negative sense strand of a target region, the probes for the positive sense strand and the probes for the negative sense strand are arranged in a non-overlapping arrangement, and a 3' or 5' end of each probe is modified with biotin which can bind to streptavidin magnetic beads.

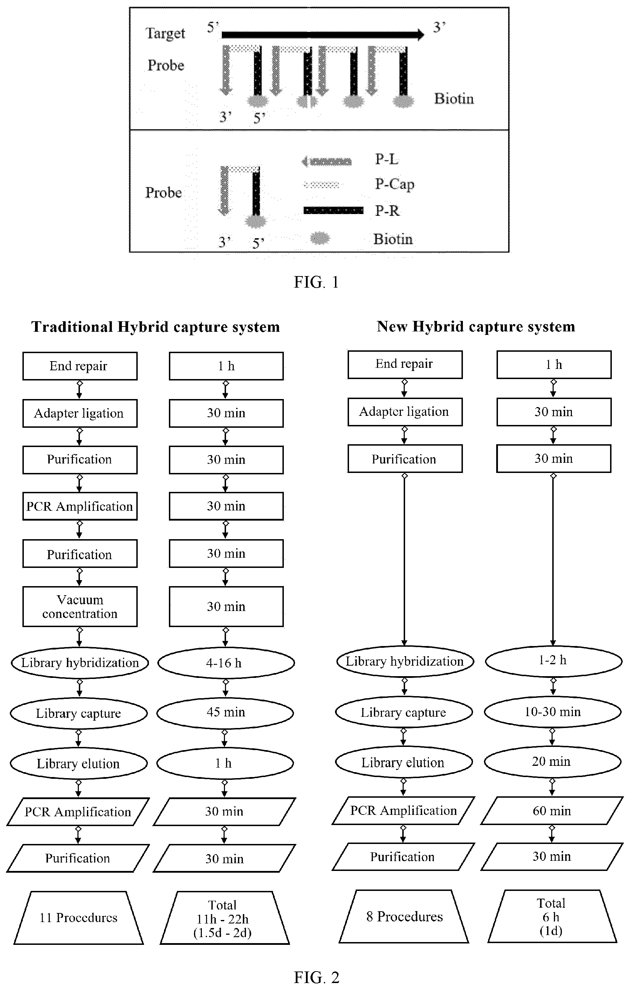

Each probe is primarily composed of three parts, wherein a middle segment is a target sequence binding segment, 5' and 3' segments are stability enhancing segments, the 5' end segment of one probe can be complementarily paired with the 3' end segment of another probe, and the 3' end segment of one probe can be complementarily paired with the 5' end segment of another probe. Fragments where the probes are complementarily paired are P-L and P-R fragments, respectively, each P-L fragment is 8-30 nt in length, each P-R fragment is 8-30 nt in length, there is a complementary pairing region of 8-30nt between the two P-L and P-R fragments, a 3' end of L or a 5' end of R is modified with biotin, and the biotin can bind to streptavidin on magnetic beads, and a fragment where the probes are complementarily paired with the target region is a P-Cap fragment with a P-Cap length of 20-80 nt (FIG. 1).

The probe design method is as follows:
probes are designed based on the positions of genes to be detected, namely if the probes are designed for mutation, insertion or deletion mutation, a region covering the corresponding fragment is selected to design the probes; and if the probes are designed for fusion genes, genes on both sides of a fusion gene breakpoint are selected to design the probes;
if capture of a sense strand is desired, a capture probe will be designed for the sense strand;
if capture of an antisense strand is desired, a capture probe will be designed for the antisense strand; and
by software analysis, hazardous probes are knocked out, and the hazardous probes will lead to severe off-target of the entire hybrid capture system, resulting in a reduced on-target rate, low target region capture efficiency, and poor coverage uniformity.

The present invention also provides a design method for a pool of probes, wherein the method is as follows:
1. probe sequence information is generated by inputting initial sequence information and design parameters through a design tool.
2. The initial sequence information includes total sequence information, i.e., information of sequences that may be contained in a library prior to enrichment, and target sequence information, i.e., information of sequences that need to be enriched from the total sequence information.
3. The design parameters include an annealing temperature range for binding a probe to a target sequence, which is related to a composition of a hybridization reaction solution and the set temperature of a reaction, and further include a length range of sequences in a region where the probes bind to a target.
4. A workflow of the design tool includes:
   (1) pre-treatment of the total sequence information. The pre-treatment of the total sequence information includes evaluating the specificity of different segments of the total sequence, and counting the number of occurrences of all combinations of sequences with a length of k in a forward strand and a complementary strand in the total sequence, wherein k is less than a minimum value of a length range of sequences in a region where the probes bind to the target sequence.
   (2) Selection of sequences in a region where the probes bind to each other. Each sequence in the region where the probes bind to each other has the characteristics including:
      (2.1) a length of k;
      (2.2) an annealing temperature that is lower than the annealing temperature for binding the probes to the target sequence; and
      (2.3) the number of occurrences being less in the total sequence, or the number of occurrences being less than 5% of the average according to the statistical result of the number of occurrences in (1) above.
   (3) Selection of the region where the probes bind to the target sequence. The selection process includes:
      (3.1) selecting an ith target sequence, i having an initial value equal to 1; and selecting sequences in a region where the probes bind to a target, starting from an nth base of the selected target sequence, n having an initial value equal to 1. Wherein the annealing temperature of the sequences in the region where the probes bind to the target satisfies 3 above, and the sequence length satisfies the range of 3 above. Wherein if the specificity of different segments in a total sequence of the sequences in the region where the probes bind to the target is evaluated as high specificity (i.e. not falling within a sequence interval that needs to be avoided), the sequence in the region where the probes bind to the target is added to the pool of probes, and m1 bases are spaced by adding a number m1 to n to try to obtain a next target specific sequence; and if the specificity of different segments in the total sequence of the sequences in the region where the probes bind to the target is evaluated as low specificity (i.e. falling within the sequence interval that needs to be avoided), the sequence in the region where the probes bind to the target is not added to the pool of probes, and m2 bases are spaced by adding a number m2 to n to try again to obtain a target specific sequence.
         Preferably, the number m1 is greater than or equal to the length of the sequence in the region where the probes bind to the target which is added to the pool of probes; and the number m2 is less than or equal to a minimum value of a length range of the sequences in the region where the probes bind to the target.
      (3.2) when n is less than a length of the ith target sequence, selecting a next sequence in the region where the probes bind to the target.
      (3.3) when n is greater than or equal to the length of the ith target sequence, finishing the selecting for the sequences in the region where the probes bind to the target of the ith target sequence.
      (3.4) after selection for the sequences in the region where the probes bind to the target of the ith target sequence is finished, performing the above selection of sequences in the region where the probes bind to the target on an i+1th target sequence until selection of sequences in the region where the probes bind to the target is completed for all target sequences.
   (4) adding a sequence in the region where the probes bind to each other to a 5' end of each sequence in the region where the probes bind to the target in the pool of probes, and adding a reverse complementary sequence of the sequence in the region where the probes bind to each other to a 3' end of each sequence in the region where the probes bind to the target in the pool of probes.
   (5) Outputting all probe sequences.

The disclosure also provides a system for construction of a target library from a nucleic acid sample (see FIG. 2), wherein a specific process is as follows:
the nucleic acid sample includes a DNA sample including plasma free DNA (cfDNA), genomic DNA (gDNA), an FFPE sample, a viral or bacterial genome sample, and the like; or a RNA sample including a fresh tissue sample, an FFPE sample, a viral or bacterial genome sample, and the like.

For the cfDNA sample, without fragmenting, library construction can be performed directly;
for a complete genome sample, physical fragmenting is needed to be performed to fragment genomic DNA to about 200-250 bp;
for the RNA sample, reverse transcription, first strand synthesis and second strand synthesis are needed to be performed; and
the fragmented sample is subjected to end repair and adapter ligation, and the ligation product is purified, and the purified product is directly subjected to hybrid capture, wherein a hybrid capture solution is related to an adapter used, multi-library mixed hybridization can be performed by using a full-UDI adapter module, and the hybridization product uses Primer Mix to perform PCR amplification on the mixed hybridization captured library; if a truncated molecular tag adaptor module is used, only a single sample can be hybridized, the molecular tag-containing adaptor module can perform low-frequency mutation detection on the sample, and hybridization ambiguity and background noise introduced by the PCR amplification are filtered out through consistent sequence analysis. Here an adapter module compatible with both Illumina and MGI sequencing platforms is used to construct a DNA library suitable for different sequencing platforms.

The adapter ligation product is directly used for configuring a hybrid capture reaction system without vacuum concentration, or hybrid capture can be performed directly with the adapter ligation product together with the purification magnetic beads from the previous step; and
a hybridization system uses specific probes designed in this project, so rapid hybridization can be performed. The hybridization time is 1-2 hours, and the capture time is 20 minutes, which shortens the hybrid capture time. The hybrid capture library is enriched by the PCR amplification. A PCR amplification solution in this step is related to the adaptor module used. The PCR amplification is performed in combination with primers containing a Barcode sequence when the molecular tag adaptor module is used, and the targetedly enriched DNA library is amplified in combination with Primer Mix if the full-length adaptor module is used (see FIG. 3).

The hybrid capture time selected for this system is 1h to 16h, with the most preferred capture time being 1h.

The hybrid capture temperature selected for this system is 59-61°C, and the optimal capture temperature is about 60°C, and temperature selection is related to a probe length, the GC content of a target region, and the hybrid capture time.

The construction of the hybrid capture library in this system takes a total of 6 hours from a sample to capture library obtaining, which greatly shortens the operation time of the whole process while simplifying the operation steps compared with the traditional 2-4 days.

The disclosure also provides hybrid capture reagent components and a use method thereof, wherein the specific content is as follows:
the adapter ligation product is purified by using 2×Beads, and the purified product is treated by using a magnetic bead wash buffer configured in a kit, wherein the magnetic bead wash buffer is 4 mL of acetonitrile added into 1 mL of H₂O.

The reagents used in the hybrid capture reaction system are detailed in Table 1.

**Table 1**

| **Reagent** | **Brand** |
|---|---|
| 2×Hyb Buffer | |
| 0.01-1% BSA | Sigma |
| 0.01-1% Ficoll | Sigma |
| 0.01-1% PVP-2 | Sigma |
| 0.01-0.5 M sodium citrate | Sigma |
| 0.1-10 M NaCl | Invitrogen |
| Enhancer: 5×formamide solution | Thermo |
| Human Cot-1 1µg/µL | Thermo |
| Blocker 100 nmol | Nanodigmbio |
| pH 6.0-8.0 | |
| Probe concentration: 2-10 fmol | Nanodigmbio |

A hybridization system involves a total of 3 elution buffers, which are an elution buffer I, an elution buffer II and an elution buffer III, respectively, and formulas of the three elution buffers are shown in Table 2.

**Table 2**

| | |
|---|---|
| Elution buffer I | 5×SSPE (Sigma), 1% of SDS (Sigma) |
| Elution buffer II | 2× SSPE, 0.1% of SDS |
| Elution buffer III | 0.1×SSPE, 0.01% of SDS |

A structural schematic diagram of the probe of the present invention, a conventional probe of 120 nt and a short probe is shown in FIG. 4. The effect of the probes of the present invention is compared with the effect of a probe commonly used in the prior art in the following Examples 1-3, and the probes of the present invention are referred to NC probes.

### Example 1: Comparison of a hybrid capture effect of a conventional probe of 120 bases with that of a short probe (not being a part of the present invention)

In this example, a library before capture is a human plasma free DNA library derived from fragmentation and release of human genomic DNA into a blood circulation system, i.e., a total sequence is the entire human genomic sequence. The target sequences given are located in the regions shown in Table 3, containing a series of high-frequency somatic mutation sites associated with tumors.

**Table 3 Locations of target sequences on hg19 version of human genome**

| **Chromosome** | **Target region starting point coordinate** | **Target region endpoint coordinate** | **Gene name** |
|---|---|---|---|
| chr1 | 115252204 | 115252205 | NRAS |
| chr1 | 115256518 | 115256533 | NRAS |
| chr1 | 115258730 | 115258752 | NRAS |
| chr2 | 209113106 | 209113193 | IDH1 |
| chr12 | 25378561 | 25378563 | KRAS |
| chr12 | 25378647 | 25378648 | KRAS |
| chr12 | 25380275 | 25380286 | KRAS |
| chr12 | 25398255 | 25398296 | KRAS |
| chr12 | 112888139 | 112888212 | PTPN11 |
| chr12 | 112926852 | 112926909 | PTPN11 |
| chr13 | 28592620 | 28592654 | FLT3 |
| chr13 | 28602329 | 28602330 | FLT3 |
| chr13 | 28608244 | 28608342 | FLT3 |
| chr13 | 28610138 | 28610139 | FLT3 |
| chr15 | 90631837 | 90631939 | IDH2 |
| chr17 | 7573931 | 7574027 | TP53 |
| chr17 | 7577022 | 7577146 | TP53 |
| chr17 | 7577515 | 7577606 | TP53 |
| chr17 | 7578187 | 7578293 | TP53 |
| chr17 | 7578362 | 7578559 | TP53 |
| chr17 | 7579358 | 7579474 | TP53 |
| chr17 | 7579882 | 7579883 | TP53 |

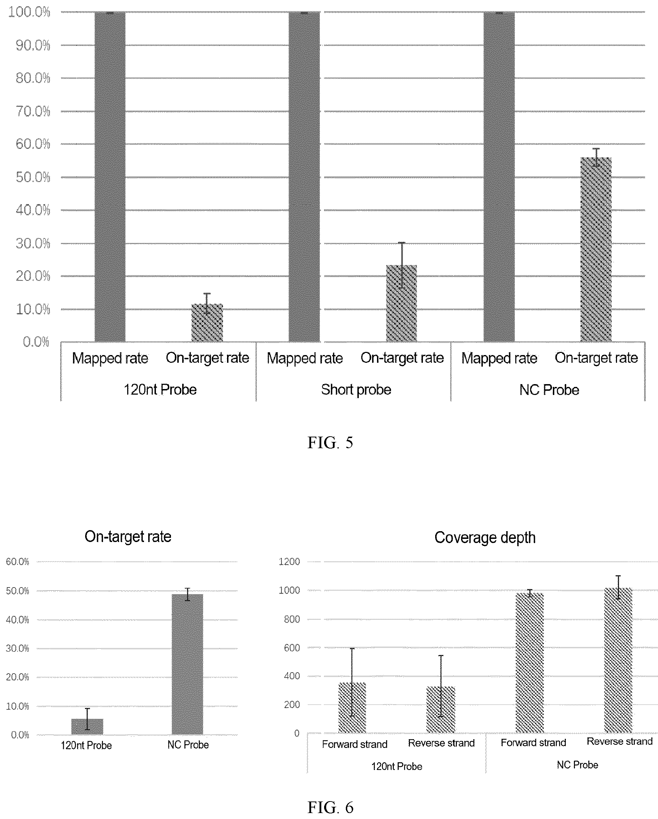

The total length of the target sequence is only 1.2 kb, and if coverage is conducted with a conventional probe of 120 nt, 44 probes are required, wherein the 44 conventional probes of 120 nt are shown in Table 4. Hybrid capture is performed with NadPrep^{®} hybrid capture reagents in this experiment, and the resulting capture library is sequenced on an Illumina Novaseq6000. In the sequencing data, 99.9% of the sequences can be mapped to a human reference genome on average, wherein 11.7% of the sequences is located in the target region on average, and the on-target rate of the probes of 120 nt is too low to meet the requirements (FIG. 5).

**Table 4 Conventional probes of 120 nt covering the target region in Table 3 (not being a part of the present invention)**

| | **Sequence name** | **Sequence 5'-3'** | **Modifi cation** |
|---|---|---|---|
| SEQ ID NO.1 | NRAS-1 | /biotin/TGCTGAAAGCTGTACCATACCTGTCTGGTCTTGGCTG AGGTTTCAATGAATGGAATCCCGTAACTCTTGGCCAGTTCG TGGGCTTGTTTTGTATCAACTGTCCTTGTTGGCAAATCACAC | 5' biotin |
| SEQ ID NO.2 | NRAS-2 | /biotin/TTTCAATGAATGGAATCCCGTAACTCTTGGCCAGTTC GTGGGCTTGTTTTGTATCAACTGTCCTTGTTGGCAAATCACA CTTGTTTCCCACTAGCACCATAGGTACATCATCCGAGTCTT | 5' biotin |
| SEQ ID NO.3 | NRAS-3 | /biotin/GCTATTATTGATGGCAAATACACAGAGGAAGCCTTCG CCTGTCCTCATGTATTGGTCTCTCATGGCACTGTACTCTTCTT GTCCAGCTGTATCCAGTATGTCCAACAAACAGGTTTCACC | 5' biotin |
| SEQ ID NO.4 | NRAS-4 | /biotin/ATTGGTCTCTCATGGCACTGTACTCTTCTTGTCCAGCT GTATCCAGTATGTCCAACAAACAGGTTTCACCATCTATAAC CACTTGTTTTCTGTAAGAATCCTGGGGGTGTGGAGGGTAAG | 5' biotin |
| SEQ ID NO.5 | NRAS-5 | /biotin/TACCACTGGGCCTCACCTCTATGGTGGGATCATATTC ATCTACAAAGTGGTTCTGGATTAGCTGGATTGTCAGTGCGC TTTTCCCAACACCACCTGCTCCAACCACCACCAGTTTGTACT | 5' biotin |
| SEQ ID NO.6 | NRAS-6 | /biotin/CTCACCTCTATGGTGGGATCATATTCATCTACAAAGT GGTTCTGGATTAGCTGGATTGTCAGTGCGCTTTTCCCAACAC CACCTGCTCCAACCACCACCAGTTTGTACTCAGTCATTTCA | 5' biotin |
| SEQ ID NO.7 | KRAS-1 | /biotin/TTTATTTCAGTGTTACTTACCTGTCTTGTCTTTGCTGAT GTTTCAATAAAAGGAATTCCATAACTTCTTGCTAAGTCCTGA GCCTGTTTTGTGTCTACTGTTCTAGAAGGCAAATCACAT | 5' biotin |
| SEQ ID NO.8 | KRAS-2 | /biotin/TTTCAATAAAAGGAATTCCATAACTTCTTGCTAAGTC CTGAGCCTGTTTTGTGTCTACTGTTCTAGAAGGCAAATCACA TTTATTTCCTACTAGGACCATAGGTACATCTTCAGAGTCCT | 5' biotin |
| SEQ ID NO.9 | KRAS-3 | /biotin/AGCCTGTTTTGTGTCTACTGTTCTAGAAGGCAAATCA CATTTATTTCCTACTAGGACCATAGGTACATCTTCAGAGTCC TTAACTCTTTTAATTTGTTCTCTGGGAAAGAAAAAAAAGTT | 5' biotin |
| SEQ ID NO.10 | KRAS-4 | /biotin/AGTATTATTTATGGCAAATACACAAAGAAAGCCCTCC CCAGTCCTCATGTACTGGTCCCTCATTGCACTGTACTCCTCT TGACCTGCTGTGTCGAGAATATCCAAGAGACAGGTTTCTCC | 5' biotin |
| SEQ ID NO.11 | KRAS-5 | /biotin/ACTGGTCCCTCATTGCACTGTACTCCTCTTGACCTGCT GTGTCGAGAATATCCAAGAGACAGGTTTCTCCATCAATTAC TACTTGCTTCCTGTAGGAATCCTGAGAAGGGAGAAACACAG | 5' biotin |
| SEQ ID NO. 12 | KRAS-6 | /biotin/TTTACCTCTATTGTTGGATCATATTCGTCCACAAAATG ATTCTGAATTAGCTGTATCGTCAAGGCACTCTTGCCTACGCC ACCAGCTCCAACTACCACAAGTTTATATTCAGTCATTTTC | 5' biotin |
| SEQ ID NO. 13 | KRAS-7 | /biotin/GTCCACAAAATGATTCTGAATTAGCTGTATCGTCAAG GCACTCTTGCCTACGCCACCAGCTCCAACTACCACAAGTTT ATATTCAGTCATTTTCAGCAGGCCTTATAATAAAAATAATG A | 5' biotin |
| SEQ ID NO.14 | PTPN11-1 | /biotin/TTTCCAATGGACTATTTTAGAAGAAATGGAGCTGTCA CCCACATCAAGATTCAGAACACTGGTGATTACTATGACCTG TATGGAGGGGAGAAATTTGCCACTTTGGCTGAGTTGGTCCA G | 5' biotin |
| SEQ ID NO.15 | PTPN11-2 | /biotin/ACTGGTGATTACTATGACCTGTATGGAGGGGAGAAAT TTGCCACTTTGGCTGAGTTGGTCCAGTATTACATGGAACATC ACGGGCAATTAAAAGAGAAGAATGGAGATGTCATTGAGCT T | 5' biotin |
| SEQ ID NO.16 | PTPN11-3 | /biotin/TCATGATGTTTCCTTCGTAGGTGTTGACTGCGATATTG ACGTTCCCAAAACCATCCAGATGGTGCGGTCTCAGAGGTCA GGGATGGTCCAGACAGAAGCACAGTACCGATTTATCTATAT | 5' biotin |
| SEQ ID NO.17 | PTPN11-4 | /biotin/GAGGTCAGGGATGGTCCAGACAGAAGCACAGTACCG ATTTATCTATATGGCGGTCCAGCATTATATTGAAACACTACA GCGCAGGATTGAAGAAGAGCAGGTACCAGCCTGAGGGCTG GC | 5' biotin |
| SEQ ID NO.18 | FLT3-1 | /biotin/TAGGAAATAGCAGCCTCACATTGCCCCTGACAACATA GTTGGAATCACTCATGATATCTCGAGCCAATCCAAAGTCAC ATATCTTCACCACTTTCCCGTGGGTGACAAGCACGTTCCTGG | 5' biotin |
| SEQ ID NO.19 | FLT3-2 | /biotin/TTGCCCCTGACAACATAGTTGGAATCACTCATGATAT CTCGAGCCAATCCAAAGTCACATATCTTCACCACTTTCCCGT GGGTGACAAGCACGTTCCTGGCGGCCAGGTCTCTGTGAACA | 5' biotin |
| SEQ ID NO.20 | FLT3-3 | /biotin/GTTACCTGACAGTGTGCACGCCCCCAGCAGGTTCACA ATATTCTCGTGGCTTCCCAGCTGGGTCATCATCTTGAGTTCT GACATGAGTGCCTCTCTTTCAGAGCTGTCTGCTTTTTCTGT | 5' biotin |
| SEQ ID NO.21 | FLT3-4 | /biotin/CCCCCAGCAGGTTCACAATATTCTCGTGGCTTCCCAG CTGGGTCATCATCTTGAGTTCTGACATGAGTGCCTCTCTTTC AGAGCTGTCTGCTTTTTCTGTCAAAGAAAGGAGCATTAAAA | 5' biotin |
| SEQ ID NO.22 | FLT3-5 | /biotin/CATTCCATTCTTACCAAACTCTAAATTTTCTCTTGGAA ACTCCCATTTGAGATCATATTCATATTCTCTGAAATCAACGT AGAAGTACTCATTATCTGAGGAGCCGGTCACCTGTACCAT | 5' biotin |
| SEQ ID NO.23 | FLT3-6 | /biotin/CTAAATTTTCTCTTGGAAACTCCCATTTGAGATCATAT TCATATTCTCTGAAATCAACGTAGAAGTACTCATTATCTGAG GAGCCGGTCACCTGTACCATCTGTAGCTGGCTTTCATACC | 5' biotin |
| SEQ ID NO.24 | FLT3-7 | /biotin/TATTACTTGGGAGACTTGTCTGAACACTTCTTCCAGGT CCAAGATGGTAATGGGTATCCATCCGAGAAACAGGACGCCT GACTTGCCGATGCTTCTGCGAGCACTTGAGGTTTCCCTATA | 5' biotin |
| SEQ ID NO.25 | FLT3-8 | /biotin/TGAACACTTCTTCCAGGTCCAAGATGGTAATGGGTAT CCATCCGAGAAACAGGACGCCTGACTTGCCGATGCTTCTGC GAGCACTTGAGGTTTCCCTATAGAAAAGAACGTGTGAAATA A | 5' biotin |
| SEQ ID NO.26 | IDH2-1 | /biotin/CCCTCTCCACCCTGGCCTACCTGGTCGCCATGGGCGT GCCTGCCAATGGTGATGGGCTTGGTCCAGCCAGGGACTAGG CGTGGGATGTTTTTGCAGATGATGGGCTCCCGGAAGACAGT C | 5' biotin |
| SEQ ID NO.27 | IDH2-2 | /biotin/TGCCAATGGTGATGGGCTTGGTCCAGCCAGGGACTAG GCGTGGGATGTTTTTGCAGATGATGGGCTCCCGGAAGACAG TCCCCCCCAGGATGTTCCGGATAGTTCCATTGGGACTTTTCC | 5' biotin |
| SEQ ID NO.28 | TP53-1 | /biotin/CACTCACCTGGAGTGAGCCCTGCTCCCCCCTGGCTCC TTCCCAGCCTGGGCATCCTTGAGTTCCAAGGCCTCATTCAGC TCTCGGAACATCTCGAAGCGCTCACGCCCACGGATCTGCAG | 5' biotin |
| SEQ ID NO.29 | TP53-2 | /biotin/TGCTCCCCCCTGGCTCCTTCCCAGCCTGGGCATCCTTG AGTTCCAAGGCCTCATTCAGCTCTCGGAACATCTCGAAGCG CTCACGCCCACGGATCTGCAGCAACAGAGGAGGGGGAGAA G | 5' biotin |
| SEQ ID NO.30 | TP53-3 | /biotin/GTGCTCCCTGGGGGCAGCTCGTGGTGAGGCTCCCCTT TCTTGCGGAGATTCTCTTCCTCTGTGCGCCGGTCTCTCCCAG GACAGGCACAAACACGCACCTCAAAGCTGTTCCGTCCCAGT | 5' biotin |
| SEQ ID NO.31 | TP53-4 | /biotin/GTGGTGAGGCTCCCCTTTCTTGCGGAGATTCTCTTCCT CTGTGCGCCGGTCTCTCCCAGGACAGGCACAAACACGCACC TCAAAGCTGTTCCGTCCCAGTAGATTACCACTACTCAGGAT | 5' biotin |
| SEQ ID NO.32 | TP53-5 | /biotin/CTGACCTGGAGTCTTCCAGTGTGATGATGGTGAGGAT GGGCCTCCGGTTCATGCCGCCCATGCAGGAACTGTTACACA TGTAGTTGTAGTGGATGGTGGTACAGTCAGAGCCAACCTAG G | 5' biotin |
| SEQ ID NO.33 | TP53-6 | /biotin/GTGATGATGGTGAGGATGGGCCTCCGGTTCATGCCGC CCATGCAGGAACTGTTACACATGTAGTTGTAGTGGATGGTG GTACAGTCAGAGCCAACCTAGGAGATAACACAGGCCCAAG AT | 5' biotin |
| SEQ ID NO.34 | TP53-7 | /biotin/AGACCTCAGGCGGCTCATAGGGCACCACCACACTATG TCGAAAAGTGTTTCTGTCATCCAAATACTCCACACGCAAAT TTCCTTCCACTCGGATAAGATGCTGAGGAGGGGCCAGACCT A | 5' biotin |
| SEQ ID NO.35 | TP53-8 | /biotin/GGCACCACCACACTATGTCGAAAAGTGTTTCTGTCAT CCAAATACTCCACACGCAAATTTCCTTCCACTCGGATAAGA TGCTGAGGAGGGGCCAGACCTAAGAGCAATCAGTGAGGAA TC | 5' biotin |
| SEQ ID NO.36 | TP53-9 | /biotin/CTCCAGCCCCAGCTGCTCACCATCGCTATCTGAGCAG CGCTCATGGTGGGGGCAGCGCCTCACAACCTCCGTCATGTG CTGTGACTGCTTGTAGATGGCCATGGCGCGGACGCGGGTGC C | 5' biotin |
| SEQ ID NO.37 | TP53-10 | /biotin/GCCTCACAACCTCCGTCATGTGCTGTGACTGCTTGTA GATGGCCATGGCGCGGACGCGGGTGCCGGGCGGGGGTGTG GAATCAACCCACAGCTGCACAGGGCAGGTCTTGGCCAGTTG GC | 5' biotin |
| SEQ ID NO.38 | TP53-11 | /biotin/CGCGGACGCGGGTGCCGGGCGGGGGTGTGGAATCAA CCCACAGCTGCACAGGGCAGGTCTTGGCCAGTTGGCAAAAC ATCTTGTTGAGGGCAGGGGAGTACTGTAGGAAGAGGAAGG AGA | 5' biotin |
| SEQ ID NO.39 | TP53-12 | /biotin/AATGCAAGAAGCCCAGACGGAAACCGTAGCTGCCCT GGTAGGTTTTCTGGGAAGGGACAGAAGATGACAGGGGCCA GGAGGGGGCTGGTGCAGGGGCCGCCGGTGTAGGAGCTGCT GGTG | 5' biotin |
| SEQ ID NO.40 | TP53-13 | /biotin/GAAGGGACAGAAGATGACAGGGGCCAGGAGGGGGC TGGTGCAGGGGCCGCCGGTGTAGGAGCTGCTGGTGCAGGG GCCACGGGGGGAGCAGCCTCTGGCATTCTGGGAGCTTCATC TGGA | 5' biotin |
| SEQ ID NO.41 | TP53-14 | /biotin/GCCCTTCCAATGGATCCACTCACAGTTTCCATAGGTC TGAAAATGTTTCCTGACTCAGAGGGGGCTCGACGCTAGGAT CTGACTGCGGCTCCTCCATGGCAGTGACCCGGAAGGCAGTC T | 5' biotin |
| SEQ ID NO.42 | TP53-15 | /biotin/CACAGTTTCCATAGGTCTGAAAATGTTTCCTGACTCA GAGGGGGCTCGACGCTAGGATCTGACTGCGGCTCCTCCATG GCAGTGACCCGGAAGGCAGTCTGGCTGCTGCAAGAGGAAA AG | 5' biotin |
| SEQ ID NO.43 | IDH1-1 | /biotin/TTATTGCCAACATGACTTACTTGATCCCCATAAGCAT GACGACCTATGATGATAGGTTTTACCCATCCACTCACAAGC CGGGGGATATTTTTGCAGATAATGGCTTCTCTGAAGACCGT G | 5' biotin |
| SEQ ID NO.44 | IDH1-2 | /biotin/AGGTTTTACCCATCCACTCACAAGCCGGGGGATATTT TTGCAGATAATGGCTTCTCTGAAGACCGTGCCACCCAGAAT ATTTCGTATGGTGCCATTTGGTGATTTCCACATTTGTTTCAA | 5' biotin |

The most concentrated length distribution of plasma free DNA fragments is about 160bp, so there is not necessarily a probe capable of binding to a plasma free DNA fragment completely, and the overall binding of the probes to the target sequence is not stable. Furthermore, the proportion of the target region to the whole genome is very small, only about 1/2500000, and thus, a low on-target rate result can also be expected.

To increase the probability of binding each fragment to probes, short probes are employed for capture. This example is intended to utilize four shorter probes (i.e., the probe length does not exceed 40 nt) for binding to each fragment to be enriched of 160 bp. The target annealing temperature of each probe is set at 65°C. The annealing temperature is greatly influenced by a sequence base composition if the probe length is shorter, so the design method for the pool of probes is different from that of the conventional probes of 120 nt, and it is necessary to adjust the probe length within a certain range to make its annealing temperature close to a target value. Design of the pool of probes is performed according to part of the steps of the design method for the pool of probes provided by the present invention (skipping the steps (1), (2) and (4) in 4), the total sequence is the human reference genome hg19, the target sequences are the target region sequences as shown in Table 3, and a probe length range parameter of 35-40 nt, and the probe annealing temperature of 65°C are input, m1 is set to be 40, and m2 is set to be 5. The resulting short probes are shown in Table 5, approximately 40 nt in length, with a total of 97 probes. After capture library NGS data analysis, it is shown that 99.9% of the sequences can be mapped to the human reference genome on average, wherein 23.4% of the sequences is located in the target region on average. Although there is a significant increase in the on-target rate, the on-target rate is still less than the requirement of conventional hybrid capture on the on-target rate of 50%. It is obvious that even if the probes are shortened directly, and the probe density is increased in overlapping probes for capture, the on-target rate cannot reach the basic requirement of 50%.

**Table 5 Short probes covering the target region in Table 3 (not being a part of the present invention)**

| | **Sequence name** | **Sequence 5'-3'** | **Modific ation** |
|---|---|---|---|
| SEQ ID NO.45 | NRAS-S-1 | /biotin/CAAATGCTGAAAGCTGTACCATACCTGTCTGG TCT | 5' biotin |
| SEQ ID NO.46 | NRAS-S-2 | /biotin/GCTGAGGTTTCAATGAATGGAATCCCGTAACT CTT | 5' biotin |
| SEQ ID NO.47 | NRAS-S-3 | /biotin/CCAGTTCGTGGGCTTGTTTTGTATCAACTGTC CTT | 5' biotin |
| SEQ ID NO.48 | NRAS-S-4 | /biotin/TGGCAAATCACACTTGTTTCCCACTAGCACCA TAG | 5' biotin |
| SEQ ID NO.49 | NRAS-S-5 | /biotin/ACATCATCCGAGTCTTTTACTCGCTTAATCTG CTC | 5' biotin |
| SEQ ID NO.50 | NRAS-S-6 | /biotin/ACTTGCTATTATTGATGGCAAATACACAGAGG AAGCC | 5' biotin |
| SEQ ID NO.51 | NRAS-S-7 | /biotin/CGCCTGTCCTCATGTATTGGTCTCTCATGGCA CTG | 5' biotin |
| SEQ ID NO.52 | NRAS-S-8 | /biotin/CTCTTCTTGTCCAGCTGTATCCAGTATGTCCA ACA | 5' biotin |
| SEQ ID NO.53 | NRAS-S-9 | /biotin/CAGGTTTCACCATCTATAACCACTTGTTTTCTG TAAGAAT | 5' biotin |
| SEQ ID NO.54 | NRAS-S-10 | /biotin/CCTGGGGGTGTGGAGGGTAAGGGGGCAGGGA GGGA | 5' biotin |
| SEQ ID NO.55 | NRAS-S-11 | /biotin/GGGCTACCACTGGGCCTCACCTCTATGGTGGG ATC | 5' biotin |
| SEQ ID NO.56 | NRAS-S-12 | /biotin/ATTCATCTACAAAGTGGTTCTGGATTAGCTGG ATTGTC | 5' biotin |
| SEQ ID NO.57 | NRAS-S-13 | /biotin/TGCGCTTTTCCCAACACCACCTGCTCCAACCA CCA | 5' biotin |
| SEQ ID NO.58 | NRAS-S-14 | /biotin/AGTTTGTACTCAGTCATTTCACACCAGCAAGA ACC | 5' biotin |
| SEQ ID NO.59 | KRAS-S-1 | /biotin/TATTTATTTCAGTGTTACTTACCTGTCTTGTCT TTGCTGA | 5' biotin |
| SEQ ID NO.60 | KRAS-S-2 | /biotin/TGTTTCAATAAAAGGAATTCCATAACTTCTTG CTAAGTCC | 5' biotin |
| SEQ ID NO.61 | KRAS-S-3 | /biotin/TGAGCCTGTTTTGTGTCTACTGTTCTAGAAGG CAA | 5' biotin |
| SEQ ID NO.62 | KRAS-S-4 | /biotin/CACATTTATTTCCTACTAGGACCATAGGTACA TCTTCAG | 5' biotin |
| SEQ ID NO.63 | KRAS-S-5 | /biotin/GTCCTTAACTCTTTTAATTTGTTCTCTGGGAAA GAAAAAA | 5' biotin |
| SEQ ID NO.64 | KRAS-S-6 | /biotin/AAGTTATAGCACAGTCATTAGTAACACAAAT ATCTTTCAA | 5' biotin |
| SEQ ID NO.65 | KRAS-S-7 | /biotin/TAGTATTATTTATGGCAAATACACAAAGAAA GCCCTCCCC | 5' biotin |
| SEQ ID NO.66 | KRAS-S-8 | /biotin/AGTCCTCATGTACTGGTCCCTCATTGCACTGT ACT | 5' biotin |
| SEQ ID NO.67 | KRAS-S-9 | /biotin/TCTTGACCTGCTGTGTCGAGAATATCCAAGAG ACA | 5' biotin |
| SEQ ID NO.68 | KRAS-S-10 | /biotin/TTTCTCCATCAATTACTACTTGCTTCCTGTAGG AATCC | 5' biotin |
| SEQ ID NO.69 | KRAS-S-11 | /biotin/AGAAGGGAGAAACACAGTCTGGATTATTACA GTGC | 5' biotin |
| SEQ ID NO.70 | KRAS-S-12 | /biotin/GATTTACCTCTATTGTTGGATCATATTCGTCCA CAAAATG | 5' biotin |
| SEQ ID NO.71 | KRAS-S-13 | /biotin/ATTCTGAATTAGCTGTATCGTCAAGGCACTCT TGC | 5' biotin |
| SEQ ID NO.72 | KRAS-S-14 | /biotin/ACGCCACCAGCTCCAACTACCACAAGTTTATA TTC | 5' biotin |
| SEQ ID NO.73 | KRAS-S-15 | /biotin/TCATTTTCAGCAGGCCTTATAATAAAAATAAT GAAAATGT | 5' biotin |
| SEQ ID NO.74 | PTPN11-S-1 | /biotin/CTTTCCAATGGACTATTTTAGAAGAAATGGAG CTGTCAC | 5' biotin |
| SEQ ID NO.75 | PTPN11-S-2 | /biotin/CACATCAAGATTCAGAACACTGGTGATTACTA TGACC | 5' biotin |
| SEQ ID NO.76 | PTPN11-S-3 | /biotin/TATGGAGGGGAGAAATTTGCCACTTTGGCTGA GTT | 5' biotin |
| SEQ ID NO.77 | PTPN 11-S-4 | /biotin/TCCAGTATTACATGGAACATCACGGGCAATTA AAAGAG | 5' biotin |
| SEQ ID NO.78 | PTPN11-S-5 | /biotin/GAATGGAGATGTCATTGAGCTTAAATATCCTC TGAACTG | 5' biotin |
| SEQ ID NO.79 | PTPN11-S-6 | /biotin/CTTCATGATGTTTCCTTCGTAGGTGTTGACTGC GA | 5' biotin |
| SEQ ID NO.80 | PTPN11-S-7 | /biotin/TTGACGTTCCCAAAACCATCCAGATGGTGCGG TCT | 5' biotin |
| SEQ ID NO.81 | PTPN11-S-8 | /biotin/GTACCGATTTATCTATATGGCGGTCCAGCATT ATATTG | 5' biotin |
| SEQ ID NO.82 | PTPN11-S-9 | /biotin/ACACTACAGCGCAGGATTGAAGAAGAGCAGG TACC | 5' biotin |
| SEQ ID NO.83 | PTPN11-S-1 0 | /biotin/CCTGAGGGCTGGCATGCGGATTCTCATTCTCT TGC | 5' biotin |
| SEQ ID NO.84 | FLT3-S-1 | /biotin/TAAGTAGGAAATAGCAGCCTCACATTGCCCCT GAC | 5' biotin |
| SEQ ID NO.85 | FLT3-S-2 | /biotin/CATAGTTGGAATCACTCATGATATCTCGAGCC AATC | 5' biotin |
| SEQ ID NO.86 | FLT3-S-3 | /biotin/AAGTCACATATCTTCACCACTTTCCCGTGGGT GAC | 5' biotin |
| SEQ ID NO.87 | FLT3-S-4 | /biotin/GCACGTTCCTGGCGGCCAGGTCTCTGTGAACA CAC | 5' biotin |
| SEQ ID NO.88 | FLT3-S-5 | /biotin/GTGGGTTACCTGACAGTGTGCACGCCCCCAGC AGG | 5' biotin |
| SEQ ID NO.89 | FLT3-S-6 | /biotin/CACAATATTCTCGTGGCTTCCCAGCTGGGTCA TCA | 5' biotin |
| SEQ ID NO.90 | FLT3-S-7 | /biotin/TTGAGTTCTGACATGAGTGCCTCTCTTTCAGA GCT | 5' biotin |
| SEQ ID NO.91 | FLT3-S-8 | /biotin/CTGCTTTTTCTGTCAAAGAAAGGAGCATTAAA AATGTAAA | 5' biotin |
| SEQ ID NO.92 | FLT3-S-9 | /biotin/GGCACATTCCATTCTTACCAAACTCTAAATTT TCTCTTGG | 5' biotin |
| SEQ ID NO.93 | FLT3-S-10 | /biotin/AAACTCCCATTTGAGATCATATTCATATTCTC TGAAATCA | 5' biotin |
| SEQ ID NO.94 | FLT3-S-11 | /biotin/ACGTAGAAGTACTCATTATCTGAGGAGCCGGT CAC | 5' biotin |
| SEQ ID NO.95 | FLT3-S-12 | /biotin/GTACCATCTGTAGCTGGCTTTCATACCTAAAT TGC | 5' biotin |
| SEQ ID NO.96 | FLT3-S-13 | /biotin/TATTACTTGGGAGACTTGTCTGAACACTTCTT CCAG | 5' biotin |
| SEQ ID NO.97 | FLT3-S-14 | /biotin/CCAAGATGGTAATGGGTATCCATCCGAGAAA CAGG | 5' biotin |
| SEQ ID NO.98 | FLT3-S-15 | /biotin/GCCTGACTTGCCGATGCTTCTGCGAGCACTTG AGG | 5' biotin |
| SEQ ID NO.99 | FLT3-S-16 | /biotin/TCCCTATAGAAAAGAACGTGTGAAATAAGCT CACTGG | 5' biotin |
| SEQ ID NO.100 | IDH2-S-1 | /biotin/ATCCCCTCTCCACCCTGGCCTACCTGGTCGCC ATG | 5' biotin |
| SEQ ID NO.101 | IDH2-S-2 | /biotin/CGTGCCTGCCAATGGTGATGGGCTTGGTCCAG CCA | 5' biotin |
| SEQ ID NO.102 | IDH2-S-3 | /biotin/GACTAGGCGTGGGATGTTTTTGCAGATGATGG GCT | 5' biotin |
| SEQ ID NO.103 | IDH2-S-4 | /biotin/CGGAAGACAGTCCCCCCCAGGATGTTCCGGA TAGT | 5' biotin |
| SEQ ID NO.104 | IDH2-S-5 | /biotin/CATTGGGACTTTTCCACATCTTCTTCAGCTTGA AC | 5' biotin |
| SEQ ID NO.105 | TP53-S-1 | /biotin/AGGTCACTCACCTGGAGTGAGCCCTGCTCCCC CCT | 5' biotin |
| SEQ ID NO.106 | TP53-S-2 | /biotin/CTCCTTCCCAGCCTGGGCATCCTTGAGTTCCA AGG | 5' biotin |
| SEQ ID NO.107 | TP53-S-3 | /biotin/TCATTCAGCTCTCGGAACATCTCGAAGCGCTC ACG | 5' biotin |
| SEQ ID NO.108 | TP53-S-4 | /biotin/CACGGATCTGCAGCAACAGAGGAGGGGGAGA AGTA | 5' biotin |
| SEQ ID NO.109 | TP53-S-5 | /biotin/AGTGCTCCCTGGGGGCAGCTCGTGGTGAGGCT CCC | 5' biotin |
| SEQ ID NO.110 | TP53-S-6 | /biotin/TTCTTGCGGAGATTCTCTTCCTCTGTGCGCCG GTC | 5' biotin |
| SEQ ID NO.111 | TP53-S-7 | /biotin/TCCCAGGACAGGCACAAACACGCACCTCAAA GCTG | 5' biotin |
| SEQ ID NO.112 | TP53-S-8 | /biotin/CCGTCCCAGTAGATTACCACTACTCAGGATAG GAA | 5' biotin |
| SEQ ID NO.113 | TP53-S-9 | /biotin/CTCCTGACCTGGAGTCTTCCAGTGTGATGATG GTG | 5' biotin |
| SEQ ID NO.114 | TP53-S-10 | /biotin/GATGGGCCTCCGGTTCATGCCGCCCATGCAGG AAC | 5' biotin |
| SEQ ID NO.115 | TP53-S-11 | /biotin/TTACACATGTAGTTGTAGTGGATGGTGGTACA GTC | 5' biotin |
| SEQ ID NO.116 | TP53-S-12 | /biotin/AGCCAACCTAGGAGATAACACAGGCCCAAGA TGAG | 5' biotin |
| SEQ ID NO.117 | TP53-S-13 | /biotin/CCAGACCTCAGGCGGCTCATAGGGCACCACC ACAC | 5' biotin |
| SEQ ID NO.118 | TP53-S-14 | /biotin/TGTCGAAAAGTGTTTCTGTCATCCAAATACTC CACAC | 5' biotin |
| SEQ ID NO.119 | TP53-S-15 | /biotin/AAATTTCCTTCCACTCGGATAAGATGCTGAGG AGG | 5' biotin |
| SEQ ID NO.120 | TP53-S-16 | /biotin/CCAGACCTAAGAGCAATCAGTGAGGAATCAG AGGC | 5' biotin |
| SEQ ID NO.121 | TP53-S-17 | /biotin/CTCCAGCCCCAGCTGCTCACCATCGCTATCTG AGC | 5' biotin |
| SEQ ID NO.122 | TP53-S-18 | /biotin/CGCTCATGGTGGGGGCAGCGCCTCACAACCTC CGT | 5' biotin |
| SEQ ID NO.123 | TP53-S-19 | /biotin/TGTGCTGTGACTGCTTGTAGATGGCCATGGCG CGG | 5' biotin |
| SEQ ID NO.124 | TP53-S-20 | /biotin/GCGGGTGCCGGCGGGGTGTGGAATCAACC CACA | 5' biotin |
| SEQ ID NO.125 | TP53-S-21 | /biotin/TGCACAGGGCAGGTCTTGGCCAGTTGGCAAA ACAT | 5' biotin |
| SEQ ID NO.126 | TP53-S-22 | /biotin/TGTTGAGGGCAGGGGAGTACTGTAGGAAGAG GAAG | 5' biotin |
| SEQ ID NO.127 | TP53-S-23 | /biotin/GACAGAGTTGAAAGTCAGGCACAAGTGAAC AGAT | 5' biotin |
| SEQ ID NO.128 | TP53-S-24 | /biotin/AATGCAAGAAGCCCAGACGGAAACCGTAGCT GCCC | 5' biotin |
| SEQ ID NO.129 | TP53-S-25 | /biotin/GTAGGTTTCTGGAAGGACAGAAGATGAC AGGG | 5' biotin |
| SEQ ID NO.130 | TP53-S-26 | /biotin/CAGGAGGGGGCTGGTGCAGGGGCCGCCGGTG TAGG | 5' biotin |
| SEQ ID NO.131 | TP53-S-27 | /biotin/CTGCTGGTGCAGGGGCCACGGGGGGAGCAGC CTCT | 5' biotin |
| SEQ ID NO.132 | TP53-S-28 | /biotin/CATTCTGGGAGCTTCATCTGGACCTGGGTCTT CAG | 5' biotin |
| SEQ ID NO.133 | TP53-S-29 | /biotin/GCCCTTCCAATGGATCCACTCACAGTTTCCAT AGG | 5' biotin |
| SEQ ID NO.134 | TP53-S-30 | /biotin/TGAAAATGTTTCCTGACTCAGAGGGGGCTCGA CGC | 5' biotin |
| SEQ ID NO.135 | TP53-S-31 | /biotin/GGATCTGACTGCGGCTCCTCCATGGCAGTGAC CCG | 5' biotin |
| SEQ ID NO.136 | TP53-S-32 | /biotin/AGGCAGTCTGGCTGCTGCAAGAGGAAAAGTG GGGA | 5' biotin |
| SEQ ID NO.137 | IDH1-S-1 | /biotin/CATTATTGCCAACATGACTTACTTGATCCCCA TAAGC | 5' biotin |
| SEQ ID NO.138 | IDH1-S-2 | /biotin/GACGACCTATGATGATAGGTTTTACCCATCCA CTC | 5' biotin |
| SEQ ID NO.139 | IDH1-S-3 | /biotin/AAGCCGGGGGATATTTTTGCAGATAATGGCTT CTC | 5' biotin |
| SEQ ID NO.140 | IDH1-S-4 | /biotin/AAGACCGTGCCACCCAGAATATTTCGTATGGT GCC | 5' biotin |
| SEQ ID NO.141 | IDH1-S-5 | /biotin/TTGGTGATTTCCACATTTGTTTCAACTTGAACT CCTCAAC | 5' biotin |

**Example 2:** Comparison of a hybrid capture effect of conventional probes of 120 bp (not being a part of the present invention) with that of the NC probes (according to the present invention)

In this example, comparison of the capture results of a human plasma free DNA library by the NC probes and conventional probes of 120bp with the capture results of the same target region in Example 1 is shown.

The NC probes are probes in which sequences for probes binding to each other are added to the short probe sequences shown in Table 5. According to the design method for the pool of probes provided by the present invention, the total sequence is the human reference genome hg19, the target sequences are the target region sequences as shown in Table 3, the probe length range is set as 35-40 nt, and the probe annealing temperature is set as 65°C. The sequence length of a region wherein probes bind to each other is set as 8, i.e., k=8. A total of 65536 of all possible sequence combinations of 8 bases occur in the human reference genome hg19, with an average number of occurrences of 88419. From sequences with the lower number of occurrences, the selected probe binding sequence is CGTCGGTC, and its complementary sequence is GACCGACG, with a number of occurrences of 2078. This sequence is added to both sides of the probes in Table 5 as the probe binding sequence.

NC probe sequences are shown in Table 6 in which probe binding sequences are added at both ends of target specific sequences of the probes compared with Table 5, and when one fragment binds more than one probe, complementary pairing between probes through probe binding sequences can increase the robustness of probe binding.

**Table 6 NC probes covering the target region in Table 3**

| | **Sequence name** | **Sequence 5'-3'** | **Modific ation** |
|---|---|---|---|
| SEQ ID NO.142 | NRAS-NC-1 | /biotin/CGTCGGTCCAAATGCTGAAAGCTGTACCATAC CTGTCTGGTCTGACCGACG | 5' biotin |
| SEQ ID NO.143 | NRAS-NC-2 | /biotin/CGTCGGTCGCTGAGGTTTCAATGAATGGAATC CCGTAACTCTTGACCGACG | 5' biotin |
| SEQ ID NO.144 | NRAS-NC-3 | /biotin/CGTCGGTCCCAGTTCGTGGGCTTGTTTTGTATC AACTGTCCTTGACCGACG | 5' biotin |
| SEQ ID NO.145 | NRAS-NC-4 | /biotin/CGTCGGTCTGGCAAATCACACTTGTTTCCCACT AGCACCATAGGACCGACG | 5' biotin |
| SEQ ID NO.146 | NRAS-NC-5 | /biotin/CGTCGGTCACATCATCCGAGTCTTTTACTCGCT TAATCTGCTCGACCGACG | 5' biotin |
| SEQ ID NO.147 | NRAS-NC-6 | /biotin/CGTCGGTCACTTGCTATTATTGATGGCAAATAC ACAGAGGAAGCCGACCGACG | 5' biotin |
| SEQ ID NO.148 | NRAS-NC-7 | /biotin/CGTCGGTCCGCCTGTCCTCATGTATTGGTCTCT CATGGCACTGGACCGACG | 5' biotin |
| SEQ ID NO.149 | NRAS-NC-8 | /biotin/CGTCGGTCCTCTTCTTGTCCAGCTGTATCCAGT ATGTCCAACAGACCGACG | 5' biotin |
| SEQ ID NO.150 | NRAS-NC-9 | /biotin/CGTCGGTCCAGGTTTCACCATCTATAACCACTT GTTTTCTGTAAGAATGACCGACG | 5' biotin |
| SEQ ID NO.151 | NRAS-NC-10 | /biotin/CGTCGGTCCCTGGGGGTGTGGAGGGTAAGGGG GCAGGGAGGGAGACCGACG | 5' biotin |
| SEQ ID NO.152 | NRAS-NC-11 | /biotin/CGTCGGTCGGGCTACCACTGGGCCTCACCTCTA TGGTGGGATCGACCGACG | 5' biotin |
| SEQ ID NO.153 | NRAS-NC-12 | /biotin/CGTCGGTCATTCATCTACAAAGTGGTTCTGGAT TAGCTGGATTGTCGACCGACG | 5' biotin |
| SEQ ID NO.154 | NRAS-NC-13 | /biotin/CGTCGGTCTGCGCTTTTCCCAACACCACCTGCT CCAACCACCAGACCGACG | 5' biotin |
| SEQ ID NO.155 | NRAS-NC-14 | /biotin/CGTCGGTCAGTTTGTACTCAGTCATTTCACACC AGCAAGAACCGACCGACG | 5' biotin |
| SEQ ID NO.156 | KRAS-NC-1 | /biotin/CGTCGGTCTATTTATTTCAGTGTTACTTACCTG TCTTGTCTTTGCTGAGACCGACG | 5' biotin |
| SEQ ID NO.157 | KRAS-NC-2 | /biotin/CGTCGGTCTGTTTCAATAAAAGGAATTCCATA ACTTCTTGCTAAGTCCGACCGACG | 5' biotin |
| SEQ ID NO.158 | KRAS-NC-3 | /biotin/CGTCGGTCTGAGCCTGTTTTGTGTCTACTGTTC TAGAAGGCAAGACCGACG | 5' biotin |
| SEQ ID NO.159 | KRAS-NC-4 | /biotin/CGTCGGTCCACATTTATTTCCTACTAGGACCAT AGGTACATCTTCAGGACCGACG | 5' biotin |
| SEQ ID NO.160 | KRAS-NC-5 | /biotin/CGTCGGTCGTCCTTAACTCTTTTAATTTGTTCTC TGGGAAAGAAAAAAGACCGACG | 5' biotin |
| SEQ ID NO.161 | KRAS-NC-6 | /biotin/CGTCGGTCAAGTTATAGCACAGTCATTAGTAA CACAAATATCTTTCAAGACCGACG | 5' biotin |
| SEQ ID NO.162 | KRAS-NC-7 | /biotin/CGTCGGTCTAGTATTATTTATGGCAAATACACA AAGAAAGCCCTCCCCGACCGACG | 5' biotin |
| SEQ ID NO. 163 | KRAS-NC-8 | /biotin/CGTCGGTCAGTCCTCATGTACTGGTCCCTCATT GCACTGTACTGACCGACG | 5' biotin |
| SEQ ID NO.164 | KRAS-NC-9 | /biotin/CGTCGGTCTCTTGACCTGCTGTGTCGAGAATAT CCAAGAGACAGACCGACG | 5' biotin |
| SEQ ID NO.165 | KRAS-NC-10 | /biotin/CGTCGGTCTTTCTCCATCAATTACTACTTGCTT CCTGTAGGAATCCGACCGACG | 5' biotin |
| SEQ ID NO.166 | KRAS-NC-11 | /biotin/CGTCGGTCAGAAGGGAGAAACACAGTCTGGAT TATTACAGTGCGACCGACG | 5' biotin |
| SEQ ID NO.167 | KRAS-NC-12 | /biotin/CGTCGGTCGATTTACCTCTATTGTTGGATCATA TTCGTCCACAAAATGGACCGACG | 5' biotin |
| SEQ ID NO.168 | KRAS-NC-13 | /biotin/CGTCGGTCATTCTGAATTAGCTGTATCGTCAAG GCACTCTTGCGACCGACG | 5' biotin |
| SEQ ID NO.169 | KRAS-NC-14 | /biotin/CGTCGGTCACGCCACCAGCTCCAACTACCACA AGTTTATATTCGACCGACG | 5' biotin |
| SEQ ID NO.170 | KRAS-NC-15 | /biotin/CGTCGGTCTCATTTTCAGCAGGCCTTATAATAA AAATAATGAAAATGTGACCGACG | 5' biotin |
| SEQ ID NO.171 | PTPN11-NC-1 | /biotin/CGTCGGTCCTTTCCAATGGACTATTTTAGAAGA AATGGAGCTGTCACGACCGACG | 5' biotin |
| SEQ ID NO.172 | PTPN11-NC-2 | /biotin/CGTCGGTCCACATCAAGATTCAGAACACTGGT GATTACTATGACCGACCGACG | 5' biotin |
| SEQ ID NO.173 | PTPN11-NC-3 | /biotin/CGTCGGTCTATGGAGGGGAGAAATTTGCCACT TTGGCTGAGTTGACCGACG | 5' biotin |
| SEQ ID NO.174 | PTPN11-NC-4 | /biotin/CGTCGGTCTCCAGTATTACATGGAACATCACG GGCAATTAAAAGAGGACCGACG | 5' biotin |
| SEQ ID NO.175 | PTPN11-NC-5 | /biotin/CGTCGGTCGAATGGAGATGTCATTGAGCTTAA ATATCCTCTGAACTGGACCGACG | 5' biotin |
| SEQ ID NO.176 | PTPN11-NC-6 | /biotin/CGTCGGTCCTTCATGATGTTTCCTTCGTAGGTG TTGACTGCGAGACCGACG | 5' biotin |
| SEQ ID NO. 177 | PTPN11-NC-7 | /biotin/CGTCGGTCTTGACGTTCCCAAAACCATCCAGAT GGTGCGGTCTGACCGACG | 5' biotin |
| SEQ ID NO.178 | PTPN11-NC-8 | /biotin/CGTCGGTCGTACCGATTTATCTATATGGCGGTC CAGCATTATATTGGACCGACG | 5' biotin |
| SEQ ID NO.179 | PTPN11-NC-9 | /biotin/CGTCGGTCACACTACAGCGCAGGATTGAAGAA GAGCAGGTACCGACCGACG | 5' biotin |
| SEQ ID NO.180 | PTPN11-NC-1 0 | /biotin/CGTCGGTCCCTGAGGGCTGGCATGCGGATTCT CATTCTCTTGCGACCGACG | 5' biotin |
| SEQ ID NO.181 | FLT3-NC-1 | /biotin/CGTCGGTCTAAGTAGGAAATAGCAGCCTCACA TTGCCCCTGACGACCGACG | 5' biotin |
| SEQ ID NO.182 | FLT3-NC-2 | /biotin/CGTCGGTCCATAGTTGGAATCACTCATGATATC TCGAGCCAATCGACCGACG | 5' biotin |
| SEQ ID NO.183 | FLT3-NC-3 | /biotin/CGTCGGTCAAGTCACATATCTTCACCACTTTCC CGTGGGTGACGACCGACG | 5' biotin |
| SEQ ID NO.184 | FLT3-NC-4 | /biotin/CGTCGGTCGCACGTTCCTGGCGGCCAGGTCTCT GTGAACACACGACCGACG | 5' biotin |
| SEQ ID NO.185 | FLT3-NC-5 | /biotin/CGTCGGTCGTGGGTTACCTGACAGTGTGCACG CCCCCAGCAGGGACCGACG | 5' biotin |
| SEQ ID NO.186 | FLT3-NC-6 | /biotin/CGTCGGTCCACAATATTCTCGTGGCTTCCCAGC TGGGTCATCAGACCGACG | 5' biotin |
| SEQ ID NO.187 | FLT3-NC-7 | /biotin/CGTCGGTCTTGAGTTCTGACATGAGTGCCTCTC TTTCAGAGCTGACCGACG | 5' biotin |
| SEQ ID NO.188 | FLT3-NC-8 | /biotin/CGTCGGTCCTGCTTTTTCTGTCAAAGAAAGGAG CATTAAAAATGTAAAGACCGACG | 5' biotin |
| SEQ ID NO.189 | FLT3-NC-9 | /biotin/CGTCGGTCGGCACATTCCATTCTTACCAAACTC TAAATTTTCTCTTGGGACCGACG | 5' biotin |
| SEQ ID NO.190 | FLT3-NC-10 | /biotin/CGTCGGTCAAACTCCCATTTGAGATCATATTCA TATTCTCTGAAATCAGACCGACG | 5' biotin |
| SEQ ID NO.191 | FLT3-NC-11 | /biotin/CGTCGGTCACGTAGAAGTACTCATTATCTGAG GAGCCGGTCACGACCGACG | 5' biotin |
| SEQ ID NO. 192 | FLT3-NC-12 | /biotin/CGTCGGTCGTACCATCTGTAGCTGGCTTTCATA CCTAAATTGCGACCGACG | 5' biotin |
| SEQ ID NO.193 | FLT3-NC-13 | /biotin/CGTCGGTCTATTACTTGGGAGACTTGTCTGAAC ACTTCTTCCAGGACCGACG | 5' biotin |
| SEQ ID NO.194 | FLT3-NC-14 | /biotin/CGTCGGTCCCAAGATGGTAATGGGTATCCATC CGAGAAACAGGGACCGACG | 5' biotin |
| SEQ ID NO.195 | FLT3-NC-15 | /biotin/CGTCGGTCGCCTGACTTGCCGATGCTTCTGCGA GCACTTGAGGGACCGACG | 5' biotin |
| SEQ ID NO. 196 | FLT3-NC-16 | /biotin/CGTCGGTCTCCCTATAGAAAAGAACGTGTGAA ATAAGCTCACTGGGACCGACG | 5' biotin |
| SEQ ID NO.197 | IDH2-NC-1 | /biotin/CGTCGGTCATCCCCTCTCCACCCTGGCCTACCT GGTCGCCATGGACCGACG | 5' biotin |
| SEQ ID NO.198 | IDH2-NC-2 | /biotin/CGTCGGTCCGTGCCTGCCAATGGTGATGGGCTT GGTCCAGCCAGACCGACG | 5' biotin |
| SEQ ID NO.199 | IDH2-NC-3 | /biotin/CGTCGGTCGACTAGGCGTGGGATGTTTTTGCA GATGATGGGCTGACCGACG | 5' biotin |
| SEQ ID NO.200 | IDH2-NC-4 | /biotin/CGTCGGTCCGGAAGACAGTCCCCCCCAGGATG TTCCGGATAGTGACCGACG | 5' biotin |
| SEQ ID NO.201 | IDH2-NC-5 | /biotin/CGTCGGTCCATTGGGACTTTTCCACATCTTCTT CAGCTTGAACGACCGACG | 5' biotin |
| SEQ ID NO.202 | TP53-NC-1 | /biotin/CGTCGGTCAGGTCACTCACCTGGAGTGAGCCC TGCTCCCCCCTGACCGACG | 5' biotin |
| SEQ ID NO.203 | TP53-NC-2 | /biotin/CGTCGGTCCTCCTTCCCAGCCTGGGCATCCTTG AGTTCCAAGGGACCGACG | 5' biotin |
| SEQ ID NO.204 | TP53-NC-3 | /biotin/CGTCGGTCTCATTCAGCTCTCGGAACATCTCGA AGCGCTCACGGACCGACG | 5' biotin |
| SEQ ID NO.205 | TP53-NC-4 | /biotin/CGTCGGTCCACGGATCTGCAGCAACAGAGGAG GGGGAGAAGTAGACCGACG | 5' biotin |
| SEQ ID NO.206 | TP53-NC-5 | /biotin/CGTCGGTCAGTGCTCCCTGGGGGCAGCTCGTG GTGAGGCTCCCGACCGACG | 5' biotin |
| SEQ ID NO.207 | TP53-NC-6 | /biotin/CGTCGGTCTTCTTGCGGAGATTCTCTTCCTCTG TGCGCCGGTCGACCGACG | 5' biotin |
| SEQ ID NO.208 | TP53-NC-7 | /biotin/CGTCGGTCTCCCAGGACAGGCACAAACACGCA CCTCAAAGCTGGACCGACG | 5' biotin |
| SEQ ID NO.209 | TP53-NC-8 | /biotin/CGTCGGTCCCGTCCCAGTAGATTACCACTACTC AGGATAGGAAGACCGACG | 5' biotin |
| SEQ ID NO.210 | TP53-NC-9 | /biotin/CGTCGGTCCTCCTGACCTGGAGTCTTCCAGTGT GATGATGGTGGACCGACG | 5' biotin |
| SEQ ID NO.211 | TP53-NC-10 | /biotin/CGTCGGTCGATGGGCCTCCGGTTCATGCCGCCC ATGCAGGAACGACCGACG | 5' biotin |
| SEQ ID NO.212 | TP53-NC-11 | /biotin/CGTCGGTCTTACACATGTAGTTGTAGTGGATGG TGGTACAGTCGACCGACG | 5' biotin |
| SEQ ID NO.213 | TP53-NC-12 | /biotin/CGTCGGTCAGCCAACCTAGGAGATAACACAGG CCCAAGATGAGGACCGACG | 5' biotin |
| SEQ ID NO.214 | TP53-NC-13 | /biotin/CGTCGGTCCCAGACCTCAGGCGGCTCATAGGG CACCACCACACGACCGACG | 5' biotin |
| SEQ ID NO.215 | TP53-NC-14 | /biotin/CGTCGGTCTGTCGAAAAGTGTTTCTGTCATCCA AATACTCCACACGACCGACG | 5' biotin |
| SEQ ID NO.216 | TP53-NC-15 | /biotin/CGTCGGTCAAATTTCCTTCCACTCGGATAAGAT GCTGAGGAGGGACCGACG | 5' biotin |
| SEQ ID NO.217 | TP53-NC-16 | /biotin/CGTCGGTCCCAGACCTAAGAGCAATCAGTGAG GAATCAGAGGCGACCGACG | 5' biotin |
| SEQ ID NO.218 | TP53-NC-17 | /biotin/CGTCGGTCCTCCAGCCCCAGCTGCTCACCATCG CTATCTGAGCGACCGACG | 5' biotin |
| SEQ ID NO.219 | TP53-NC-18 | /biotin/CGTCGGTCCGCTCATGGTGGGGGCAGCGCCTC ACAACCTCCGTGACCGACG | 5' biotin |
| SEQ ID NO.220 | TP53-NC-19 | /biotin/CGTCGGTCTGTGCTGTGACTGCTTGTAGATGGC CATGGCGCGGGACCGACG | 5' biotin |
| SEQ ID NO.221 | TP53-NC-20 | /biotin/CGTCGGTCGCGGGTGCCGGGCGGGGGTGTGGA ATCAACCCACAGACCGACG | 5' biotin |
| SEQ ID NO.222 | TP53-NC-21 | /biotin/CGTCGGTCTGCACAGGGCAGGTCTTGGCCAGT TGGCAAAACATGACCGACG | 5' biotin |
| SEQ ID NO.223 | TP53-NC-22 | /biotin/CGTCGGTCTGTTGAGGGCAGGGGAGTACTGTA GGAAGAGGAAGGACCGACG | 5' biotin |
| SEQ ID NO.224 | TP53-NC-23 | /biotin/CGTCGGTCGACAGAGTTGAAAGTCAGGGCACA AGTGAACAGATGACCGACG | 5' biotin |
| SEQ ID NO.225 | TP53-NC-24 | /biotin/CGTCGGTCAATGCAAGAAGCCCAGACGGAAAC CGTAGCTGCCCGACCGACG | 5' biotin |
| SEQ ID NO.226 | TP53-NC-25 | /biotin/CGTCGGTCGTAGGTTTTCTGGGAAGGGACAGA AGATGACAGGGGACCGACG | 5' biotin |
| SEQ ID NO.227 | TP53-NC-26 | /biotin/CGTCGGTCCAGGAGGGGGCTGGTGCAGGGGCC GCCGGTGTAGGGACCGACG | 5' biotin |
| SEQ ID NO.228 | TP53-NC-27 | /biotin/CGTCGGTCCTGCTGGTGCAGGGGCCACGGGGG GAGCAGCCTCTGACCGACG | 5' biotin |
| SEQ ID NO.229 | TP53-NC-28 | /biotin/CGTCGGTCCATTCTGGGAGCTTCATCTGGACCT GGGTCTTCAGGACCGACG | 5' biotin |
| SEQ ID NO.230 | TP53-NC-29 | /biotin/CGTCGGTCGCCCTTCCAATGGATCCACTCACAG TTTCCATAGGGACCGACG | 5' biotin |
| SEQ ID NO.231 | TP53-NC-30 | /biotin/CGTCGGTCTGAAAATGTTTCCTGACTCAGAGG GGGCTCGACGCGACCGACG | 5' biotin |
| SEQ ID NO.232 | TP53-NC-31 | /biotin/CGTCGGTCGGATCTGACTGCGGCTCCTCCATGG CAGTGACCCGGACCGACG | 5' biotin |
| SEQ ID NO.233 | TP53-NC-32 | /biotin/CGTCGGTCAGGCAGTCTGGCTGCTGCAAGAGG AAAAGTGGGGAGACCGACG | 5' biotin |
| SEQ ID NO.234 | IDH1-NC-1 | /biotin/CGTCGGTCCATTATTGCCAACATGACTTACTTG ATCCCCATAAGCGACCGACG | 5' biotin |
| SEQ ID NO.235 | IDH1-NC-2 | /biotin/CGTCGGTCGACGACCTATGATGATAGGTTTTAC CCATCCACTCGACCGACG | 5' biotin |
| SEQ ID NO.236 | IDH1-NC-3 | /biotin/CGTCGGTCAAGCCGGGGATATTTTGCAGAT AATGGCTTCTCGACCGACG | 5' biotin |
| SEQ ID NO.237 | IDH1-NC-4 | /biotin/CGTCGGTCAAGACCGTGCCACCCAGAATATTT CGTATGGTGCCGACCGACG | 5' biotin |
| SEQ ID NO.238 | IDH1-NC-5 | /biotin/CGTCGGTCTTGGTGATTTCCACATTTGTTTCAA CTTGAACTCCTCAACGACCGACG | 5' biotin |

The results are shown in FIG. 5, NGS data of the NC probe captured library shows that 99.9% of the sequences can be mapped to the human reference genome, and an average proportion of sequences located in the target regions is 56.0%, which meets the on-target rate requirements in conventional hybrid capture.

### Example 3: Targeted capture of a PCR-free library using NC probes

**A** PCR-free library refers to a library that is connected to a NGS adapter, but is not subjected to PCR amplification, wherein original sequence information is retained, and PCR preferences are not introduced. Hybrid capture with the PCR-free library directly suffers from the difficulties of low hybridization input and an unguaranteed capture rate. After PCR amplification of the library, each original fragment has multiple copies, so there are multiple opportunities to be bound and captured by probes. If any fragment in the PCR-free library is not captured by the probes, it cannot enter a next step, resulting in information loss. Moreover, after PCR, each single strand of the library fragment generates a corresponding complementary strand, so the probes only need to be designed in one direction to capture information from both strands of the original fragment. Whereas in the PCR-free library, both forward and reverse strands of one fragment are present singly, and if the probes in only one direction are used for capture, the complementary strands will also be lost. Thus, in this example, a probe of the other strand is added. The other strand probes for the conventional probes of 120 bp are shown in Table 7, and the other strand probes for the NC probes are shown in Table 8.

As shown in FIG. 6, after 30 ng of a plasma free DNA PCR-free library is captured by the conventional probes of 120 bp in Tables 4 and 7, the NGS results show an average on-target rate of only 5.6%, an average coverage depth of 356.1x of forward strands after deduplication, and an average depth of 329.9x of reverse strands after deduplication. Whereas after capture by the NC probes shown in Tables 6 and 8, the NGS results show that the average on-target rate reaches 48.7%, with an average depth of 980.2x of the forward strands after deduplication, and an average depth of 1020.5x of the reverse strands after deduplication. It can be seen that for the PCR-free library, the recovery rate and the on-target rate for the NC probes are greatly improved.

**Table 7 Complementary strand probes for the conventional probes of 120bp covering the target region in Table 3 (not being a part of the present invention)**

| | **Sequence name** | **Sequence 5'-3'** | **Modificat ion** |
|---|---|---|---|
| SEQ ID NO.239 | NRAS-OP-1 | /biotin/GTGTGATTTGCCAACAAGGACAGTTGATACA AAACAAGCCCAC GAACTGGCCAAGAGTTACGGGA TTCCATTCATTGAAACCTCAGCCAAGACCAGACAG GTATGGTACAGCTTTCAGCA | 5' biotin |
| SEQ ID NO.240 | NRAS-OP-2 | /biotin/AAGACTCGGATGATGTACCTATGGTGCTAGT GGGAAACAAGTGTGATTTGCCAACAAGGACAGTTG ATACAAAACAAGCCCACGAACTGGCCAAGAGTTAC GGGATTCCATTCATTGAAA | 5' biotin |
| SEQ ID NO.241 | NRAS-OP-3 | /biotin/GGTGAAACCTGTTTGTTGGACATACTGGATA CAGCTGGACAAGAAGAGTACAGTGCCATGAGAGA CCAATACATGAGGACAGGCGAAGGCTTCCTCTGTG TATTTGCCATCAATAATAGC | 5' biotin |
| SEQ ID NO.242 | NRAS-OP-4 | /biotin/CTTACCCTCCACACCCCCAGGATTCTTACAG AAAACAAGTGGTTATAGATGGTGAAACCTGTTTGT TGGACATACTGGATACAGCTGGACAAGAAGAGTAC AGTGCCATGAGAGACCAAT | 5' biotin |
| SEQ ID NO.243 | NRAS-OP-5 | /biotin/AGTACAAACTGGTGGTGGTTGGAGCAGGTG GTGTTGGGAAAAGCGCACTGACAATCCAGCTAATC CAGAACCACTTTGTAGATGAATATGATCCCACCAT AGAGGTGAGGCCCAGTGGTA | 5' biotin |
| SEQ ID NO.244 | NRAS-OP-6 | /biotin/TGAAATGACTGAGTACAAACTGGTGGTGGTT GGAGCAGGTGGTGTTGGGAAAAGCGCACTGACAA TCCAGCTAATCCAGAACCACTTTGTAGATGAATAT GATCCCACCATAGAGGTGAG | 5' biotin |
| SEQ ID NO.245 | KRAS-OP-1 | /biotin/ATGTGATTTGCCTTCTAGAACAGTAGACACA AAACAGGCTCAGGACTTAGCAAGAAGTTATGGAAT TCCTTTTATTGAAACATCAGCAAAGACAAGACAGG TAAGTAACACTGAAATAAA | 5' biotin |
| SEQ ID NO.246 | KRAS-OP-2 | /biotin/AGGACTCTGAAGATGTACCTATGGTCCTAGT AGGAAATAAATGTGATTTGCCTTCTAGAACAGTAG ACACAAAACAGGCTCAGGACTTAGCAAGAAGTTAT GGAATTCCTTTTATTGAAA | 5' biotin |
| SEQ ID NO.247 | KRAS-OP-3 | /biotin/AACTTTTTTCTTTCCCAGAGAACAAATTAA AAGAGTTAAGGACTCTGAAGATGTACCTATGGTCC TAGTAGGAAATAAATGTGATTTGCCTTCTAGAACA GTAGACACAAAACAGGCT | 5' biotin |
| SEQ ID NO.248 | KRAS-OP-4 | /biotin/GGAGAAACCTGTCTCTTGGATATTCTCGACA CAGCAGGTCAAGAGGAGTACAGTGCAATGAGGGA CCAGTACATGAGGACTGGGGAGGGCTTTCTTTGTG TATTTGCCATAAATAATACT | 5' biotin |
| SEQ ID NO.249 | KRAS-OP-5 | /biotin/CTGTGTTTCTCCCTTCTCAGGATTCCTACAGG AAGCAAGTAGTAATTGATGGAGAAACCTGTCTCTT GGATATTCTCGACACAGCAGGTCAAGAGGAGTACA GTGCAATGAGGGACCAGT | 5' biotin |
| SEQ ID NO.250 | KRAS-OP-6 | /biotin/GAAAATGACTGAATATAAACTTGTGGTAGTT GGAGCTGGTGGCGTAGGCAAGAGTGCCTTGACGAT ACAGCTAATTCAGAATCATTTTGTGGACGAATATG ATCCAACAATAGAGGTAAA | 5' biotin |
| SEQ ID NO.251 | KRAS-OP-7 | /biotin/TCATTATTTTTATTATAAGGCCTGCTGAAAAT GACTGAATATAAACTTGTGGTAGTTGGAGCTGGTG GCGTAGGCAAGAGTGCCTTGACGATACAGCTAATT CAGAATCATTTTGTGGAC | 5' biotin |
| SEQ ID NO.252 | PTPN11-OP-1 | /biotin/CTGGACCAACTCAGCCAAAGTGGCAAATTTC TCCCCTCCATACAGGTCATAGTAATCACCAGTGTTC TGAATCTTGATGTGGGTGACAGCTCCATTTCTTCTA AAATAGTCCATTGGAAA | 5' biotin |
| SEQ ID NO.253 | PTPN11-OP-2 | /biotin/AAGCTCAATGACATCTCCATTCTTCTCTTTTA ATTGCCCGTGATGTTCCATGTAATACTGGACCAACT CAGCCAAAGTGGCAAATTTCTCCCCTCCATACAGG TCATAGTAATCACCAGT | 5' biotin |
| SEQ ID NO.254 | PTPN11-OP-3 | /biotin/ATATAGATAAATCGGTACTGTGCTTCTGTCT GGACCATCCCTGACCTCTGAGACCGCACCATCTGG ATGGTTTTGGGAACGTCAATATCGCAGTCAACACC TACGAAGGAAACATCATGA | 5' biotin |
| SEQ ID NO.255 | PTPN11-OP-4 | /biotin/GCCAGCCCTCAGGCTGGTACCTGCTCTTCTTC AATCCTGCGCTGTAGTGTTTCAATATAATGCTGGAC CGCCATATAGATAAATCGGTACTGTGCTTCTGTCTG GACCATCCCTGACCTC | 5' biotin |
| SEQ ID NO.256 | FLT3-OP-1 | /biotin/CCAGGAACGTGCTTGTCACCCACGGGAAAGT GGTGAAGATATGTGACTTTGGATTGGCTCGAGATA TCATGAGTGATTCCAACTATGTTGTCAGGGGCAAT GTGAGGCTGCTATTTCCTA | 5' biotin |
| SEQ ID NO.257 | FLT3-OP-2 | /biotin/TGTTCACAGAGACCTGGCCGCCAGGAACGTG CTTGTCACCCACGGGAAAGTGGTGAAGATATGTGA CTTTGGATTGGCTCGAGATATCATGAGTGATTCCA ACTATGTTGTCAGGGGCAA | 5' biotin |
| SEQ ID NO.258 | FLT3-OP-3 | /biotin/ACAGAAAAAGCAGACAGCTCTGAAAGAGAG GCACTCATGTCAGAACTCAAGATGATGACCCAGCT GGGAAGCCACGAGAATATTGTGAACCTGCTGGGGG CGTGCACACTGTCAGGTAAC | 5' biotin |
| SEQ ID NO.259 | FLT3-OP-4 | /biotin/TTTTAATGCTCCTTTCTTTGACAGAAAAAGC AGACAGCTCTGAAAGAGAGGCACTCATGTCAGAAC TCAAGATGATGACCCAGCTGGGAAGCCACGAGAAT ATTGTGAACCTGCTGGGGG | 5' biotin |
| SEQ ID NO.260 | FLT3-OP-5 | /biotin/ATGGTACAGGTGACCGGCTCCTCAGATAATG AGTACTTCTACGTTGATTTCAGAGAATATGAATAT GATCTCAAATGGGAGTTTCCAAGAGAAAATTTAGA GTTTGGTAAGAATGGAATG | 5' biotin |
| SEQ ID NO.261 | FLT3-OP-6 | /biotin/GGTATGAAAGCCAGCTACAGATGGTACAGG TGACCGGCTCCTCAGATAATGAGTACTTCTACGTTG ATTTCAGAGAATATGAATATGATCTCAAATGGGAG TTTCCAAGAGAAAATTTAG | 5' biotin |
| SEQ ID NO.262 | FLT3-OP-7 | /biotin/TATAGGGAAACCTCAAGTGCTCGCAGAAGC ATCGGCAAGTCAGGCGTCCTGTTTCTCGGATGGAT ACCCATTACCATCTTGGACCTGGAAGAAGTGTTCA GACAAGTCTCCCAAGTAATA | 5' biotin |
| SEQ ID NO.263 | FLT3-OP-8 | /biotin/TTATTTCACACGTTCTTTTCTATAGGGAAACC TCAAGTGCTCGCAGAAGCATCGGCAAGTCAGGCGT CCTGTTTCTCGGATGGATACCCATTACCATCTTGGA CCTGGAAGAAGTGTTCA | 5' biotin |
| SEQ ID NO.264 | IDH2-OP-1 | /biotin/GACTGTCTTCCGGGAGCCCATCATCTGCAAA AACATCCCACGCCTAGTCCCTGGCTGGACCAAGCC CATCACCATTGGCAGGCACGCCCATGGCGACCAGG TAGGCCAGGGTGGAGAGGG | 5' biotin |
| SEQ ID NO.265 | IDH2-OP-2 | /biotin/GGAAAAGTCCCAATGGAACTATCCGGAACA TCCTGGGGGGGACTGTCTTCCGGGAGCCCATCATC TGCAAAAACATCCCACGCCTAGTCCCTGGCTGGAC CAAGCCCATCACCATTGGCA | 5' biotin |
| SEQ ID NO.266 | TP53-OP-1 | /biotin/CTGCAGATCCGTGGGCGTGAGCGCTTCGAGA TGTTCCGAGAGCTGAATGAGGCCTTGGAACTCAAG GATGCCCAGGCTGGGAAGGAGCCAGGGGGGAGCA GGGCTCACTCCAGGTGAGTG | 5' biotin |
| SEQ ID NO.267 | TP53-OP-2 | /biotin/CTTCTCCCCCTCCTCTGTTGCTGCAGATCCGT GGGCGTGAGCGCTTCGAGATGTTCCGAGAGCTGAA TGAGGCCTTGGAACTCAAGGATGCCCAGGCTGGGA AGGAGCCAGGGGGGAGCA | 5' biotin |
| SEQ ID NO.268 | TP53-OP-3 | /biotin/ACTGGGACGGAACAGCTTTGAGGTGCGTGTT TGTGCCTGTCCTGGGAGAGACCGGCGCACAGAGGA AGAGAATCTCCGCAAGAAAGGGGAGCCTCACCAC GAGCTGCCCCCAGGGAGCAC | 5' biotin |
| SEQ ID NO.269 | TP53-OP-4 | /biotin/ATCCTGAGTAGTGGTAATCTACTGGGACGGA ACAGCTTTGAGGTGCGTGTTTGTGCCTGTCCTGGGA GAGACCGGCGCACAGAGGAAGAGAATCTCCGCAA GAAAGGGGAGCCTCACCAC | 5' biotin |
| SEQ ID NO.270 | TP53-OP-5 | /biotin/CCTAGGTTGGCTCTGACTGTACCACCATCCA CTACAACTACATGTGTAACAGTTCCTGCATGGGCG GCATGAACCGGAGGCCCATCCTCACCATCATCACA CTGGAAGACTCCAGGTCAG | 5' biotin |
| SEQ ID NO.271 | TP53-OP-6 | /biotin/ATCTTGGGCCTGTGTTATCTCCTAGGTTGGCT CTGACTGTACCACCATCCACTACAACTACATGTGT AACAGTTCCTGCATGGGCGGCATGAACCGGAGGCC CATCCTCACCATCATCAC | 5' biotin |
| SEQ ID NO.272 | TP53-OP-7 | /biotin/TAGGTCTGGCCCCTCCTCAGCATCTTATCCG AGTGGAAGGAAATTTGCGTGTGGAGTATTTGGATG ACAGAAACACTTTTCGACATAGTGTGGTGGTGCCC TATGAGCCGCCTGAGGTCT | 5' biotin |
| SEQ ID NO.273 | TP53-OP-8 | /biotin/GATTCCTCACTGATTGCTCTTAGGTCTGGCCC CTCCTCAGCATCTTATCCGAGTGGAAGGAAATTTG CGTGTGGAGTATTTGGATGACAGAAACACTTTTCG ACATAGTGTGGTGGTGCC | 5' biotin |
| SEQ ID NO.274 | TP53-OP-9 | /biotin/GGCACCCGCGTCCGCGCCATGGCCATCTACA AGCAGTCACAGCACATGACGGAGGTTGTGAGGCGC TGCCCCCACCATGAGCGCTGCTCAGATAGCGATGG TGAGCAGCTGGGGCTGGAG | 5' biotin |
| SEQ ID NO.275 | TP53-OP-10 | /biotin/GCCAACTGGCCAAGACCTGCCCTGTGCAGCT GTGGGTTGATTCCACACCCCCGCCCGGCACCCGCG TCCGCGCCATGGCCATCTACAAGCAGTCACAGCAC ATGACGGAGGTTGTGAGGC | 5' biotin |
| SEQ ID NO.276 | TP53-OP-11 | /biotin/TCTCCTTCCTCTTCCTACAGTACTCCCCTGCC CTCAACAAGATGTTTTGCCAACTGGCCAAGACCTG CCCTGTGCAGCTGTGGGTTGATTCCACACCCCCGCC CGGCACCCGCGTCCGCG | 5' biotin |
| SEQ ID NO.277 | TP53-OP-12 | /biotin/CACCAGCAGCTCCTACACCGGCGGCCCCTGC ACCAGCCCCCTCCTGGCCCCTGTCATCTTCTGTCCC TTCCCAGAAAACCTACCAGGGCAGCTACGGTTTCC GTCTGGGCTTCTTGCATT | 5' biotin |
| SEQ ID NO.278 | TP53-OP-13 | /biotin/TCCAGATGAAGCTCCCAGAATGCCAGAGGCT GCTCCCCCCGTGGCCCCTGCACCAGCAGCTCCTAC ACCGGCGGCCCCTGCACCAGCCCCCTCCTGGCCCC TGTCATCTTCTGTCCCTTC | 5' biotin |
| SEQ ID NO.279 | TP53-OP-14 | /biotin/AGACTGCCTTCCGGGTCACTGCCATGGAGGA GCCGCAGTCAGATCCTAGCGTCGAGCCCCCTCTGA GTCAGGAAACATTTTCAGACCTATGGAAACTGTGA GTGGATCCATTGGAAGGGC | 5' biotin |
| SEQ ID NO.280 | TP53-OP-15 | /biotin/CTTTTCCTCTTGCAGCAGCCAGACTGCCTTCC GGGTCACTGCCATGGAGGAGCCGCAGTCAGATCCT AGCGTCGAGCCCCCTCTGAGTCAGGAAACATTTTC AGACCTATGGAAACTGTG | 5' biotin |
| SEQ ID NO.281 | IDH1-OP-1 | /biotin/CACGGTCTTCAGAGAAGCCATTATCTGCAAA AATATCCCCCGGCTTGTGAGTGGATGGGTAAAACC TATCATCATAGGTCGTCATGCTTATGGGGATCAAG TAAGTCATGTTGGCAATAA | 5' biotin |
| SEQ ID NO.282 | IDH1-OP-2 | /biotin/TTGAAACAAATGTGGAAATCACCAAATGGC ACCATACGAAATATTCTGGGTGGCACGGTCTTCAG AGAAGCCATTATCTGCAAAAATATCCCCCGGCTTG TGAGTGGATGGGTAAAACCT | 5' biotin |

**Table 8 Complementary strand probes for the NC probes covering the target region in Table 3**

| | **Sequence name** | **Sequence 5'-3'** | **Modific ation** |
|---|---|---|---|
| SEQ ID NO.283 | NRAS-NC-OP-1 | /biotin/CGTCGGTCAGACCAGACAGGTATGGTACAGCTTTC AGCATTTGGACCGACG | 5' biotin |
| SEQ ID NO.284 | NRAS-NC-OP-2 | /biotin/CGTCGGTCAAGAGTTACGGGATTCCATTCATTGAA ACCTCAGCGACCGACG | 5' biotin |
| SEQ ID NO.285 | NRAS-NC-OP-3 | /biotin/CGTCGGTCAAGGACAGTTGATACAAAACAAGCCC ACGAACTGGGACCGACG | 5' biotin |
| SEQ ID NO.286 | NRAS-NC-OP-4 | /biotin/CGTCGGTCCTATGGTGCTAGTGGGAAACAAGTGTG ATTTGCCAGACCGACG | 5' biotin |
| SEQ ID NO.287 | NRAS-NC-OP-5 | /biotin/CGTCGGTCGAGCAGATTAAGCGAGTAAAAGACTC GGATGATGTGACCGACG | 5' biotin |
| SEQ ID NO.288 | NRAS-NC-OP-6 | /biotin/CGTCGGTCGGCTTCCTCTGTGTATTTGCCATCAATA ATAGCAAGTGACCGACG | 5' biotin |
| SEQ ID NO.289 | NRAS-NC-OP-7 | /biotin/CGTCGGTCCAGTGCCATGAGAGACCAATACATGAG GACAGGCGGACCGACG | 5' biotin |
| SEQ ID NO.290 | NRAS-NC-OP-8 | /biotin/CGTCGGTCTGTTGGACATACTGGATACAGCTGGAC AAGAAGAGGACCGACG | 5' biotin |
| SEQ ID NO.291 | NRAS-NC-OP-9 | /biotin/CGTCGGTCATTCTTACAGAAAACAAGTGGTTATAG ATGGTGAAACCTGGACCGACG | 5' biotin |
| SEQ ID NO.292 | NRAS-NC-OP-10 | /biotin/CGTCGGTCTCCCTCCCTGCCCCCTTACCCTCCACAC CCCCAGGGACCGACG | 5' biotin |
| SEQ ID NO.293 | NRAS-NC-OP-11 | /biotin/CGTCGGTCGATCCCACCATAGAGGTGAGGCCCAGT GGTAGCCCGACCGACG | 5' biotin |
| SEQ ID NO.294 | NRAS-NC-OP-12 | /biotin/CGTCGGTCGACAATCCAGCTAATCCAGAACCACTT TGTAGATGAATGACCGACG | 5' biotin |
| SEQ ID NO.295 | NRAS-NC-OP-13 | /biotin/CGTCGGTCTGGTGGTTGGAGCAGGTGGTGTTGGGA AAAGCGCAGACCGACG | 5' biotin |
| SEQ ID NO.296 | NRAS-NC-OP-14 | /biotin/CGTCGGTCGGTTCTTGCTGGTGTGAAATGACTGAG TACAAACTGACCGACG | 5' biotin |
| SEQ ID NO.297 | KRAS-NC-OP-1 | /biotin/CGTCGGTCTCAGCAAAGACAAGACAGGTAAGTAA CACTGAAATAAATAGACCGACG | 5' biotin |
| SEQ ID NO.298 | KRAS-NC-OP-2 | /biotin/CGTCGGTCGGACTTAGCAAGAAGTTATGGAATTCC TTTTATTGAAACAGACCGACG | 5' biotin |
| SEQ ID NO.299 | KRAS-NC-OP-3 | /biotin/CGTCGGTCTTGCCTTCTAGAACAGTAGACACAAAA CAGGCTCAGACCGACG | 5' biotin |
| SEQ ID NO.300 | KRAS-NC-OP-4 | /biotin/CGTCGGTCCTGAAGATGTACCTATGGTCCTAGTAG GAAATAAATGTGGACCGACG | 5' biotin |
| SEQ ID NO.301 | KRAS-NC-OP-5 | /biotin/CGTCGGTCTTTTTTCTTTCCCAGAGAACAAATTAAA AGAGTTAAGGACGACCGACG | 5' biotin |
| SEQ ID NO.302 | KRAS-NC-OP-6 | /biotin/CGTCGGTCTTGAAAGATATTTGTGTTACTAATGACT GTGCTATAACTTGACCGACG | 5' biotin |
| SEQ ID NO.303 | KRAS-NC-OP-7 | /biotin/CGTCGGTCGGGAGGGCTTTCTTGTGTATTTGCCA TAAATAATACTAGACCGACG | 5' biotin |
| SEQ ID NO.304 | KRAS-NC-OP-8 | /biotin/CGTCGGTCAGTACAGTGCAATGAGGGACCAGTACA TGAGGACTGACCGACG | 5' biotin |
| SEQ ID NO.305 | KRAS-NC-OP-9 | /biotin/CGTCGGTCTGTCTCTTGGATATTCTCGACACAGCA GGTCAAGAGACCGACG | 5' biotin |
| SEQ ID NO.306 | KRAS-NC-OP-10 | /biotin/CGTCGGTCGGATTCCTACAGGAAGCAAGTAGTAAT TGATGGAGAAAGACCGACG | 5' biotin |
| SEQ ID NO.307 | KRAS-NC-OP-11 | /biotin/CGTCGGTCGCACTGTAATAATCCAGACTGTGTTTCT CCCTTCTGACCGACG | 5' biotin |
| SEQ ID NO.308 | KRAS-NC-OP-12 | /biotin/CGTCGGTCCATTTTGTGGACGAATATGATCCAACA ATAGAGGTAAATCGACCGACG | 5' biotin |
| SEQ ID NO.309 | KRAS-NC-OP-13 | /biotin/CGTCGGTCGCAAGAGTGCCTTGACGATACAGCTAA TTCAGAATGACCGACG | 5' biotin |
| SEQ ID NO.310 | KRAS-NC-OP-14 | /biotin/CGTCGGTCGAATATAAACTTGTGGTAGTTGGAGCT GGTGGCGTGACCGACG | 5' biotin |
| SEQ ID NO.311 | KRAS-NC-OP-15 | /biotin/CGTCGGTCACATTTTCATTATTTTTATTATAAGGCC TGCTGAAAATGAGACCGACG | 5' biotin |
| SEQ ID NO.312 | PTPN11-N C-OP-1 | /biotin/CGTCGGTCGTGACAGCTCCATTTCTTCTAAAATAGT CCATTGGAAAGGACCGACG | 5' biotin |
| SEQ ID NO.313 | PTPN11-N C-OP-2 | /biotin/CGTCGGTCGGTCATAGTAATCACCAGTGTTCTGAA TCTTGATGTGGACCGACG | 5' biotin |
| SEQ ID NO.314 | PTPN11-N C-OP-3 | /biotin/CGTCGGTCAACTCAGCCAAAGTGGCAAATTTCTCC CCTCCATAGACCGACG | 5' biotin |
| SEQ ID NO.315 | PTPN11-N C-OP-4 | /biotin/CGTCGGTCCTCTTTTAATTGCCCGTGATGTTCCATG TAATACTGGAGACCGACG | 5' biotin |
| SEQ ID NO.316 | PTPN11-N C-OP-5 | /biotin/CGTCGGTCCAGTTCAGAGGATATTTAAGCTCAATG ACATCTCCATTCGACCGACG | 5' biotin |
| SEQ ID NO.317 | PTPN11-N C-OP-6 | /biotin/CGTCGGTCTCGCAGTCAACACCTACGAAGGAAACA TCATGAAGGACCGACG | 5' biotin |
| SEQ ID NO.318 | PTPN11-N C-OP-7 | /biotin/CGTCGGTCAGACCGCACCATCTGGATGGTTTTGGG AACGTCAAGACCGACG | 5' biotin |
| SEQ ID NO.319 | PTPN11-N C-OP-8 | /biotin/CGTCGGTCCAATATAATGCTGGACCGCCATATAGA TAAATCGGTACGACCGACG | 5' biotin |
| SEQ ID NO.320 | PTPN11-N C-OP-9 | /biotin/CGTCGGTCGGTACCTGCTCTTCTTCAATCCTGCGCT GTAGTGTGACCGACG | 5' biotin |
| SEQ ID NO.321 | PTPN11-N C-OP-10 | /biotin/CGTCGGTCGCAAGAGAATGAGAATCCGCATGCCA GCCCTCAGGGACCGACG | 5' biotin |
| SEQ ID NO.322 | FLT3-NC-O P-1 | /biotin/CGTCGGTCGTCAGGGGCAATGTGAGGCTGCTATTT CCTACTTAGACCGACG | 5' biotin |
| SEQ ID NO.323 | FLT3-NC-O P-2 | /biotin/CGTCGGTCGATTGGCTCGAGATATCATGAGTGATT CCAACTATGGACCGACG | 5' biotin |
| SEQ ID NO.324 | FLT3-NC-O P-3 | /biotin/CGTCGGTCGTCACCCACGGGAAAGTGGTGAAGATA TGTGACTTGACCGACG | 5' biotin |
| SEQ ID NO.325 | FLT3-NC-O P-4 | /biotin/CGTCGGTCGTGTGTTCACAGAGACCTGGCCGCCAG GAACGTGCGACCGACG | 5' biotin |
| SEQ ID NO.326 | FLT3-NC-O P-5 | /biotin/CGTCGGTCCCTGCTGGGGGCGTGCACACTGTCAGG TAACCCACGACCGACG | 5' biotin |
| SEQ ID NO.327 | FLT3-NC-O P-6 | /biotin/CGTCGGTCTGATGACCCAGCTGGGAAGCCACGAGA ATATTGTGGACCGACG | 5' biotin |
| SEQ ID NO.328 | FLT3-NC-O P-7 | /biotin/CGTCGGTCAGCTCTGAAAGAGAGGCACTCATGTCA GAACTCAAGACCGACG | 5' biotin |
| SEQ ID NO.329 | FLT3-NC-O P-8 | /biotin/CGTCGGTCTTTACATTTTTAATGCTCCTTTCTTTGA CAGAAAAAGCAGGACCGACG | 5' biotin |
| SEQ ID NO.330 | FLT3-NC-O P-9 | /biotin/CGTCGGTCCCAAGAGAAAATTTAGAGTTTGGTAAG AATGGAATGTGCCGACCGACG | 5' biotin |
| SEQ ID NO.331 | FLT3-NC-O P-10 | /biotin/CGTCGGTCTGATTTCAGAGAATATGAATATGATCT CAAATGGGAGTTTGACCGACG | 5' biotin |
| SEQ ID NO.332 | FLT3-NC-O P-11 | /biotin/CGTCGGTCGTGACCGGCTCCTCAGATAATGAGTAC TTCTACGTGACCGACG | 5' biotin |
| SEQ ID NO.333 | FLT3-NC-O P-12 | /biotin/CGTCGGTCGCAATTTAGGTATGAAAGCCAGCTACA GATGGTACGACCGACG | 5' biotin |
| SEQ ID NO.334 | FLT3-NC-O P-13 | /biotin/CGTCGGTCCTGGAAGAAGTGTTCAGACAAGTCTCC CAAGTAATAGACCGACG | 5' biotin |
| SEQ ID NO.335 | FLT3-NC-O P-14 | /biotin/CGTCGGTCCCTGTTTCTCGGATGGATACCCATTACC ATCTTGGGACCGACG | 5' biotin |
| SEQ ID NO.336 | FLT3-NC-O P-15 | /biotin/CGTCGGTCCCTCAAGTGCTCGCAGAAGCATCGGCA AGTCAGGCGACCGACG | 5' biotin |
| SEQ ID NO.337 | FLT3-NC-O P-16 | /biotin/CGTCGGTCCCAGTGAGCTTATTTCACACGTTCTTTT CTATAGGGAGACCGACG | 5' biotin |
| SEQ ID NO.338 | IDH2-NC-O P-1 | /biotin/CGTCGGTCCATGGCGACCAGGTAGGCCAGGGTGG AGAGGGGATGACCGACG | 5' biotin |
| SEQ ID NO.339 | IDH2-NC-O P-2 | /biotin/CGTCGGTCTGGCTGGACCAAGCCCATCACCATTGG CAGGCACGGACCGACG | 5' biotin |
| SEQ ID NO.340 | IDH2-NC-O P-3 | /biotin/CGTCGGTCAGCCCATCATCTGCAAAAACATCCCAC GCCTAGTCGACCGACG | 5' biotin |
| SEQ ID NO.341 | IDH2-NC-O P-4 | /biotin/CGTCGGTCACTATCCGGAACATCCTGGGGGGGACT GTCTTCCGGACCGACG | 5' biotin |
| SEQ ID NO.342 | IDH2-NC-O P-5 | /biotin/CGTCGGTCGTTCAAGCTGAAGAAGATGTGGAAAA GTCCCAATGGACCGACG | 5' biotin |
| SEQ ID NO.343 | TP53-NC-O P-1 | /biotin/CGTCGGTCAGGGGGGAGCAGGGCTCACTCCAGGT GAGTGACCTGACCGACG | 5' biotin |
| SEQ ID NO.344 | TP53-NC-O P-2 | /biotin/CGTCGGTCCCTTGGAACTCAAGGATGCCCAGGCTG GGAAGGAGGACCGACG | 5' biotin |
| SEQ ID NO.345 | TP53-NC-O P-3 | /biotin/CGTCGGTCCGTGAGCGCTTCGAGATGTTCCGAGAG CTGAATGAGACCGACG | 5' biotin |
| SEQ ID NO.346 | TP53-NC-O P-4 | /biotin/CGTCGGTCTACTTCTCCCCCTCCTCTGTTGCTGCAG ATCCGTGGACCGACG | 5' biotin |
| SEQ ID NO.347 | TP53-NC-O P-5 | /biotin/CGTCGGTCGGGAGCCTCACCACGAGCTGCCCCCAG GGAGCACTGACCGACG | 5' biotin |
| SEQ ID NO.348 | TP53-NC-O P-6 | /biotin/CGTCGGTCGACCGGCGCACAGAGGAAGAGAATCT CCGCAAGAAGACCGACG | 5' biotin |
| SEQ ID NO.349 | TP53-NC-O P-7 | /biotin/CGTCGGTCCAGCTTTGAGGTGCGTGTTTGTGCCTGT CCTGGGAGACCGACG | 5' biotin |
| SEQ ID NO.350 | TP53-NC-O P-8 | /biotin/CGTCGGTCTTCCTATCCTGAGTAGTGGTAATCTACT GGGACGGGACCGACG | 5' biotin |
| SEQ ID NO.351 | TP53-NC-O P-9 | /biotin/CGTCGGTCCACCATCATCACACTGGAAGACTCCAG GTCAGGAGGACCGACG | 5' biotin |
| SEQ ID NO.352 | TP53-NC-O P-10 | /biotin/CGTCGGTCGTTCCTGCATGGGCGGCATGAACCGGA GGCCCATCGACCGACG | 5' biotin |
| SEQ ID NO.353 | TP53-NC-O P-11 | /biotin/CGTCGGTCGACTGTACCACCATCCACTACAACTAC ATGTGTAAGACCGACG | 5' biotin |
| SEQ ID NO.354 | TP53-NC-O P-12 | /biotin/CGTCGGTCCTCATCTTGGGCCTGTGTTATCTCCTAG GTTGGCTGACCGACG | 5' biotin |
| SEQ ID NO.355 | TP53-NC-O P-13 | /biotin/CGTCGGTCGTGTGGTGGTGCCCTATGAGCCGCCTG AGGTCTGGGACCGACG | 5' biotin |
| SEQ ID NO.356 | TP53-NC-O P-14 | /biotin/CGTCGGTCGTGTGGAGTATTTGGATGACAGAAACA CTTTTCGACAGACCGACG | 5' biotin |
| SEQ ID NO.357 | TP53-NC-O P-15 | /biotin/CGTCGGTCCCTCCTCAGCATCTTATCCGAGTGGAA GGAAATTTGACCGACG | 5' biotin |
| SEQ ID NO.358 | TP53-NC-O P-16 | /biotin/CGTCGGTCGCCTCTGATTCCTCACTGATTGCTCTTA GGTCTGGGACCGACG | 5' biotin |
| SEQ ID NO.359 | TP53-NC-O P-17 | /biotin/CGTCGGTCGCTCAGATAGCGATGGTGAGCAGCTGG GGCTGGAGGACCGACG | 5' biotin |
| SEQ ID NO.360 | TP53-NC-O P-18 | /biotin/CGTCGGTCACGGAGGTTGTGAGGCGCTGCCCCCAC CATGAGCGGACCGACG | 5' biotin |
| SEQ ID NO.361 | TP53-NC-O P-19 | /biotin/CGTCGGTCCCGCGCCATGGCCATCTACAAGCAGTC ACAGCACAGACCGACG | 5' biotin |
| SEQ ID NO.362 | TP53-NC-O P-20 | /biotin/CGTCGGTCTGTGGGTTGATTCCACACCCCCGCCCG GCACCCGCGACCGACG | 5' biotin |
| SEQ ID NO.363 | TP53-NC-O P-21 | /biotin/CGTCGGTCATGTTTTGCCAACTGGCCAAGACCTGC CCTGTGCAGACCGACG | 5' biotin |
| SEQ ID NO.364 | TP53-NC-O P-22 | /biotin/CGTCGGTCCTTCCTCTTCCTACAGTACTCCCCTGCC CTCAACAGACCGACG | 5' biotin |
| SEQ ID NO.365 | TP53-NC-O P-23 | /biotin/CGTCGGTCATCTGTTCACTTGTGCCCTGACTTTCAA CTCTGTCGACCGACG | 5' biotin |
| SEQ ID NO.366 | TP53-NC-O P-24 | /biotin/CGTCGGTCGGGCAGCTACGGTTTCCGTCTGGGCTT CTTGCATTGACCGACG | 5' biotin |
| SEQ ID NO.367 | TP53-NC-O P-25 | /biotin/CGTCGGTCCCCTGTCATCTTCTGTCCCTTCCCAGAA AACCTACGACCGACG | 5' biotin |
| SEQ ID NO.368 | TP53-NC-O P-26 | /biotin/CGTCGGTCCCTACACCGGCGGCCCCTGCACCAGCC CCCTCCTGGACCGACG | 5' biotin |
| SEQ ID NO.369 | TP53-NC-O P-27 | /biotin/CGTCGGTCAGAGGCTGCTCCCCCCGTGGCCCCTGC ACCAGCAGGACCGACG | 5' biotin |
| SEQ ID NO.370 | TP53-NC-O P-28 | /biotin/CGTCGGTCCTGAAGACCCAGGTCCAGATGAAGCTC CCAGAATGGACCGACG | 5' biotin |
| SEQ ID NO.371 | TP53-NC-O P-29 | /biotin/CGTCGGTCCCTATGGAAACTGTGAGTGGATCCATT GGAAGGGCGACCGACG | 5' biotin |
| SEQ ID NO.372 | TP53-NC-O P-30 | /biotin/CGTCGGTCGCGTCGAGCCCCCTCTGAGTCAGGAAA CATTTTCAGACCGACG | 5' biotin |
| SEQ ID NO.373 | TP53-NC-O P-31 | /biotin/CGTCGGTCCGGGTCACTGCCATGGAGGAGCCGCAG TCAGATCCGACCGACG | 5' biotin |
| SEQ ID NO.374 | TP53-NC-O P-32 | /biotin/CGTCGGTCTCCCCACTTTTCCTCTTGCAGCAGCCAG ACTGCCTGACCGACG | 5' biotin |
| SEQ ID NO.375 | IDH1-NC-O P-1 | /biotin/CGTCGGTCGCTTATGGGGATCAAGTAAGTCATGTT GGCAATAATGGACCGACG | 5' biotin |
| SEQ ID NO. 376 | IDH1-NC-O P-2 | /biotin/CGTCGGTCGAGTGGATGGGTAAAACCTATCATCAT AGGTCGTCGACCGACG | 5' biotin |
| SEQ ID NO. 377 | IDH1-NC-O P-3 | /biotin/CGTCGGTCGAGAAGCCATTATCTGCAAAAATATCC CCCGGCTTGACCGACG | 5' biotin |
| SEQ ID NO.378 | IDH1-NC-O P-4 | /biotin/CGTCGGTCGGCACCATACGAAATATTCTGGGTGGC ACGGTCTTGACCGACG | 5' biotin |
| SEQ ID NO.379 | IDH1-NC-O P-5 | /biotin/CGTCGGTCGTTGAGGAGTTCAAGTTGAAACAAATG TGGAAATCACCAAGACCGACG | 5' biotin |

After testing the basic effect of the NC probes of the present invention, Examples 4-8 further test the hybrid capture system based on the NC probes of the present invention and related parameters.

### Example 4: Optimal NC probe concentration test

The difference in capture efficiency of NC probes with different concentrations for target genes is unknown, and through an experiment in which probes of different concentration gradients are set, the optimal probe concentration is sought. A specific experimental protocol is shown in Table 9 below, and a target region of 4.5 kb is designed according to the probe design concept of the present invention, and a Promega standard male (G1471 Promega-male) is used to fragment a sample to about 200-250 bp.

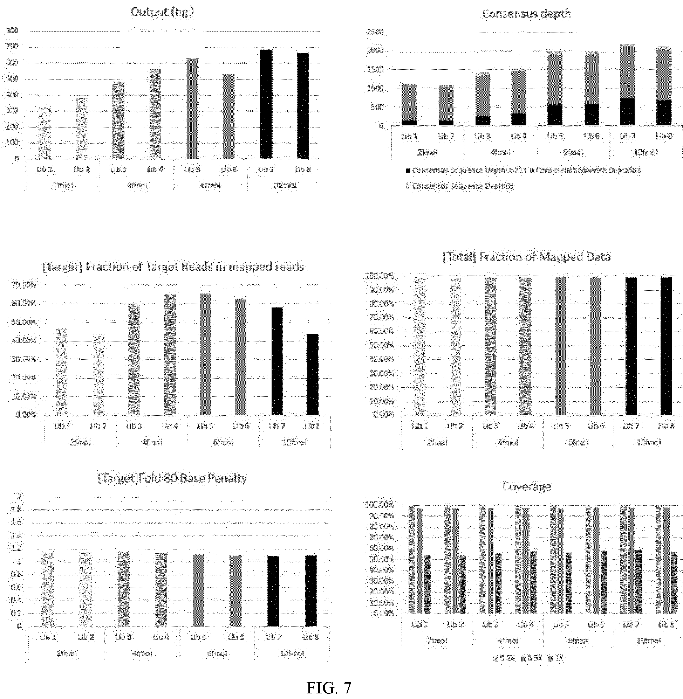

For a specific experimental process, other variables are consistent except for different probe concentrations in experimental groups. Result data is shown in FIG. 7.

**Table 9**

| **Experimental group** | **Probe concentration** |
|---|---|
| Lib 1 | 2 fmol |
| Lib 2 | 2 fmol |
| Lib 3 | 4 fmol |
| Lib 4 | 4 fmol |
| Lib 5 | 6 fmol |
| Lib 6 | 6 fmol |
| Lib 7 | 10 fmol |
| Lib 8 | 10 fmol |

From result analysis of the Consensus depth, DS211 or SS information is directly proportional to the NC probe concentration. When the NC probe concentration is lower, less effective library information is captured, the higher the NC probe concentration, the richer the captured effective library information, but a too high NC probe concentration will lead to an excess of redundant NC probes in the system, resulting in a decrease in on-target rate. The optimal NC probe concentration used in this system is 6-10 fmol, with 6 fmol of the NC probe being more preferred.

### Example 5: Optimal hybrid capture temperature test

This system uses the NC probes, and the hybrid capture temperature needs to be selected according to the probe structure. In order to determine the optimal temperature conditions, a series of tests are performed. A specific experimental protocol is shown in Table 10 below. A target region of 4.5 kb is designed according to the design concept of the NC probes of the present invention. A Promega standard male (G1471 Promega-male) is used to fragment a sample to about 200-250 bp.

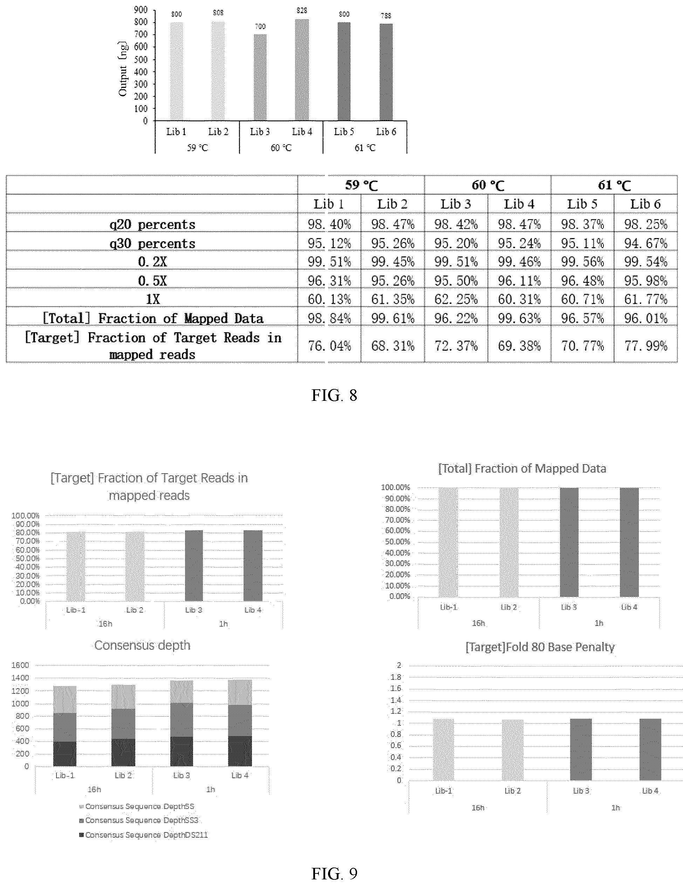

For a specific experimental process, other variables are consistent except for different hybrid capture temperatures in experimental groups. Result data is shown in FIG. 8.

**Table 10**

| **Experimental group** | **Hybrid capture temperature** |
|---|---|
| Lib 1 | 57°C |
| Lib 2 | 60°C |
| Lib 3 | 63°C |

From result analysis of the library construction efficiency and the Consensus depth, the DS211 or SS content is affected by the hybrid capture temperature, the hybrid capture temperature of 60°C performs better than the other two temperature conditions, and the capture efficiency and the on-target rate at the hybrid capture temperature of 60°C are higher than those at the other hybrid capture temperatures.

To ensure that 60°C is the optimal hybridization condition, and that this system is not too sensitive to the hybridization temperature, closer hybridization conditions are then tested to compare the difference in library capture efficiency under hybridization conditions of 59°C, 60°C, and 61°C (see Table 11), other variables are consistent except for different hybrid capture temperatures in experimental groups, and the result data is shown in FIG. 8.

**Table 11**

| **Experimental group** | **Hybrid capture temperature** |
|---|---|
| Lib 1 | 59°C |
| Lib 2 | 60°C |
| Lib 3 | 61°C |

From the above data analysis, hybridization temperatures from 59°C to 61°C show a superior capture efficiency, with 60°C being used as the final hybrid capture condition for this system.

### Example 6: Shortening hybrid capture time

The hybridization time used in a traditional hybrid capture system is 16 hours, while the hybridization time used in the present invention can be reduced from 16 hours to 1 hour, and shortening the hybridization time does not affect the efficiency of the probes in capturing DNA samples.

An experiment is carried out by using the hybrid capture conditions of this system, a specific experimental protocol is shown in Table 12 below, a target region of 50 kb is first designed according to the design idea of the NC probes of the present invention, and a GW-OGTM800 standard is used to fragment a sample to about 200-250 bp.

The experimental process is as follows:
gDNA is fragmented to about 200 bp (Covaris ultrasonic fragmentation instrument), and end repair and adapter ligation are carried out, followed by purification of nucleic acid using an equal volume of Beads; and this specific purification process is as follows:
1. NadPrep^{®} SP Beads are taken out in advance, vortexing is conducted for uniform mixing, and equilibration is conducted at room temperature for 30 minutes before use;
2. 80µL of NadPrep^{®} SP Beads is added to the adapter ligation product to be uniformly mixed, and the mixture is incubated at 25°C for 5-10 minutes;
3. a PCR tube is instantaneously centrifuged, and placed on a magnetic rack for 5-10 minutes until liquid is completely clear, and a supernatant is discarded by pipetting with a pipette;
4. 200µL of BW Buffer is added for washing once, the washed material is allowed to stand for 2 minutes, and a supernatant is discarded by pipetting; and
5. a hybridization reaction solution is added to the reaction system.

A hybridization system contains 6 fmol of probe, 1×Hyb Buffer, 1×Enhance, 1µg of Human Cot-1, and 100 pmmol of Blocker, and the configured hybridization reaction system is placed in a temperature controller for a reaction under the following conditions: denaturation at 95°C for 2 minutes, and hybridization at 60°C for 1 hour or 16 hours.

After completion of the hybridization reaction, the supernatant is transferred to a new PCR tube, and 10µL of M270 Beads is added to the PCR reaction tube for hybrid capture at 60°C for 20 minutes.

After the end of 20 minutes of capture, washing is separately performed once with an elution buffer I, an elution buffer II, and an elution buffer III.

After washing is completed, a PCR reaction system is added to the M270 Beads, wherein the PCR reaction system mainly includes 2×HiFi PCR Master Mix, 5µL of Index Primer Mix, and 20µL of TE; a PCR amplification procedure is started on a PCR temperature controller, and after the reaction is finished, the resulting product is purified by using 1×magnetic beads, and the purified product is sequenced on an Illumina^{®} platform.

Test result data is shown in FIG. 9.

**Table 12**

| **Experimental group** | **Library construction and hybrid capture kit** | **Hybridization time** |
|---|---|---|
| Lib 1 | EASY Hybrid Capture System | 16 hours |
| Lib 2 | EASY Hybrid Capture System | 16 hours |
| Lib 3 | EASY Hybrid Capture System | 1 hour |
| Lib 4 | EASY Hybrid Capture System | 1 hour |

From result analysis of the Consensus depth, DS211 or SS information is directly proportional to the hybridization time, 90% or more of the efficient library have been captured after 1 hour of hybridization, with the final selection of the hybridization time of 1 hour, and the entire experimental process is controlled to be completed in one day.

### Example 7: Comparison of capture of a small target region by the NC probes in a PCR-free mode with a conventional capture process with conventional probes

In order to compare the performance of capture with NC probes in an optimized PCR-free mode with that of capture with traditional probes in a non-PCR-free mode for a small target region, an experiment is performed according to a grouping method in Table 13 below, wherein a group 1 uses a traditional manner to construct a targeted capture library, with the traditional hybrid capture system matched with probes of 120 nt; and a group 2 uses a system of the NC probes of the present invention to construct a PCR-free targeted capture library, capture probes are designed for a same region, the probes cover genomic exon regions, and the target region size is about 4 kb.

**Table 13**

| **Experimental group** | **Library construction kit** | **Hybrid capture kit** | |
|---|---|---|---|
| Group 1 | NadPrep DNA universal library construction kit | NadPrep^{®} Hybrid Capture Reagents | Control group |
| Group 2 | EASY Hybrid Capture System | EASY Hybrid Capture System | Experimental group |

Wherein a specific implementation process in the group 1 refers to a commercial instruction for a NadPrep^{®} simple hybrid capture kit; while a specific experimental process in the group 2 refers to that in Example 6, and the hybridization time is fixed at 1 hour.

The data performance of this example is shown in Table 14. The mean coverage in the group 1 and the group 2 is close to 100%, while the on-target rate in the group 2 is 59%, which is higher than 11.73% in the group 1. It is obvious that the system of the NC probes of the present invention can effectively improve the on-target rate.

**Table 14. Small target region capture efficiency higher than traditional hybrid capture**

| | **Group 1 Traditional** | **Group 2 EASY Cap** |
|---|---|---|
| Fraction of Target Reads in mapped reads | 11.73% | 59% |
| Fraction of Mapped Reads | 99.29% | 99.32% |
| 0.2×Mean coverage | 100.00% | 100% |
| 0.5×Mean coverage | 98.92% | 100% |
| Fold 80 base penalty | 1.17 | 1.12 |
| Note: | | |
| • [Target] Fraction of Target Reads in mapped reads: a proportion of target reads in mapped reads. | | |
| • Fraction of Mapped reads: a proportion of reads mapped to a human genome in all reads. | | |
| • 0.2×Mean Coverage: 0.2×mean coverage percentage. | | |
| • 0.5×Mean coverage: 0.5×mean coverage percentage. | | |
| • Fold 80 Base Penalty: sequencing multiples required to be increased to ensure that 80% of target bases reaches the original average coverage depth. | | |

### Example 8: Detection efficiency of fusion genes higher than traditional hybrid capture

Fusion genes are produced when partial fragments of two genes are joined due to genome rearrangement. The fusion genes can be detected and analyzed by capturing and sequencing regions on both sides of a rearrangement breakpoint. Due to the fact that only part of rearrangement fragments across the breakpoint is the original sequence, for conventional probes, there may be a problem where only part of the fragments can be bound. The NC probes can also improve the detection ability of fusion genes through more probe binding possibilities.

An experiment is carried out according to a grouping method Table 15 below, wherein Group 1 uses a conventional manner to construct a targeted capture library, with a traditional hybrid capture system matched with probes of 120nt, and probes covering the ROS1 intron 33 are designed to detect CD74-ROS1 fusion; and Group 2 uses the present invention to construct a targeted capture library, with capture probes designed for the same region, a target region of about 1 kb. Wherein a specific implementation process in the group 1 refers to the commercial instruction for the NadPrep^{®} simple hybrid capture kit.

**Table 15**

| **Experimental group** | **Library construction kit** | **Hybrid capture kit** | |
|---|---|---|---|
| Group 1 | NadPrep DNA universal library construction kit | NadPrep^{®} Hybrid Capture Reagents | Control group |
| Group 2 | EASY Hybrid Capture System | EASY Hybrid Capture System | Experimental group |

The sample is a pan-tumor 800 gDNA standard (GW-OGTM800) containing multiple digital PCR verified mutation sites, one of which is CD74-ROS1 Fusion, and this site has a theoretical mutation frequency of 6%.

The specific experimental process in the group 2 refers to that in Example 6, and result data is shown in Table 16 below.

**Table 16 Detection efficiency of fusion genes higher than traditional hybrid capture**

| | **Group 1 Traditional** | **Group 2 EASY Cap** |
|---|---|---|
| Fraction of Target Reads in mapped reads | 10.5% | 52.3% |
| Fraction of Mapped Reads | 98.08% | 99.88% |
| 0.2×Mean coverage | 98.46% | 100.00% |
| 0.5×Mean coverage | 89.57% | 88.24% |
| CD74-ROS1 (a theoretical value of 6%) | 1.1% | 5.8% |
| Note: | | |
| • [Target] Fraction of Target Reads in mapped reads: a proportion of target reads in mapped reads. | | |
| • Fraction of Mapped reads: a proportion of reads mapped to a human genome in all reads. | | |
| • 0.2×Mean Coverage: 0.2×mean coverage percentage. | | |
| • 0.5×Mean coverage: 0.5×mean coverage percentage. | | |
| • Fold 80 Base Penalty: sequencing multiples required to be increased to ensure that 80% of target bases reaches the original average coverage depth. | | |

Fusion sites are often located within a repeating region, and a probe design within the repeating region is something of a capture challenge. However, the use of the NC probes in this system shows certain advantages for the detection of the fusion genes. The GW-OGTM800 standard in this experiment contains a set of CD74-ROS1 fusion genes with a mutation frequency of 5% as verified by digital PCR; and the Group 1 and the Group 2 use probes covering the same region for hybrid capture, and the frequency of detecting fusion genes by the traditional method is about 1.1%, while the frequency of detecting fusion genes by the optimized system of the present invention is 5.8%.

## Claims

1. A set of probes for nucleic acid hybridization capture, comprising multiple different probes, wherein each probe comprises
a middle segment which is a respective target specific sequence complementarily pairing with a corresponding nucleic acid target sequence,
a first probe binding sequence, which is located at 5' end of the probe and is complementarily pairing with a 3' end of another probe,
and
a second probe binding sequence, which is located at 3' end of the probe and is complementarily pairing with a 5' end of another probe;
wherein the first probe binding sequence is 8-30 nt in length, the second probe binding sequence is 8-30 nt in length,
wherein the respective target specific sequence is 20-80 nt in length;
wherein the 3' end or the 5' end of each probe has a biomarker;
wherein the biomarker is biotin for binding to streptavidin on magnetic beads;
wherein the annealing temperature between each probe and respective nucleic acid target sequence is greater than the annealing temperature between probes.

2. A method for obtaining a set of probes for nucleic acid hybridization capture according to claim 1, wherein the method is performed by a design tool, and the method comprise following steps of:
step a, inputting initial sequence information and design parameters, and outputting probe sequence information, wherein the initial sequence information comprises
total sequence information, which is information of sequences that are possible to be contained before capture in a library; and
target sequence information, which is information of sequences to be captured, and sequences that need to be avoided, which are low-specificity sequences such as repeated sequences in a comprehensive sequence;
wherein the design parameters comprise an annealing temperature range and a sequence length range for binding a probe to a target sequence, and a length range of a probe binding sequence;
step b, slicing out all subsequences with a length of k, wherein k is 20-80 nt, from forward strand sequences and complementary strand sequences in the total sequence, and counting the number of occurrences of each subsequence, and obtaining an average number of occurrences;
step c, selecting a probe binding sequence in which probes are complementary paired, wherein the probe binding sequence has a length of k, wherein k is 8-30 nt, and has an annealing temperature that is lower than the annealing temperature for binding the probe to the target sequence, and the occurrence of the probe binding sequence in the total sequence is less than 5% of the average number of occurrences;
step d, selecting target specific sequences in which each probe binds to a respective nucleic acid target sequence: firstly, an ith target sequence is selected, i having an initial value equal to 1; and then the respective target specific sequence in which the probe binds to the ith target sequence is then selected, starting from an nth base of the selected target sequence, n having an initial value equal to 1;
step e, adding the probe binding sequence to a 5' end of each target specific sequence, and adding a reverse complementary sequence of the probe binding sequence to a 3' end of each target specific sequence; and
step f, outputting all probe sequences.

3. Use of the set of probes according to claim 1 for low-frequency mutation detection, chromosome copy number variation analysis, insertion/deletion, microsatellite instability or fusion gene variation in DNA fragments.

4. Use of the probe according to claim 1 for targeted metagenomic next-generation sequencing, mNGS, or detection of pathogen epidemiology.

## Patentansprüche

1. Satz von Sonden zur Capture-Hybridisierung von Nukleinsäuren, umfassend mehrere verschiedene Sonden, wobei jede Sonde Folgendes umfasst:
ein mittleres Segment, das eine jeweilige zielspezifische Sequenz ist, die komplementär ein Paar mit einer entsprechenden Nukleinsäurezielsequenz bildet,
eine erste Sondenbindungssequenz, die sich an einem 5'-Ende der Sonde befindet und komplementär ein Paar mit einem 3'-Ende einer anderen Sonde bildet,
und
eine zweite Sondenbindungssequenz, die sich an einem 3'-Ende der Sonde befindet und komplementär ein Paar mit einem 5'-Ende einer anderen Sonde bildet;
wobei die erste Sondenbindungssequenz 8-30 nt lang ist, die zweite Sondenbindungssequenz 8-30 nt lang ist,
wobei die jeweilige zielspezifische Sequenz 20-80 nt lang ist;
wobei das 3'-Ende oder das 5'-Ende jeder Sonde einen Biomarker aufweist;
wobei der Biomarker Biotin zum Binden an Streptavidin auf magnetischen Kügelchen ist;
wobei die Annealing-Temperatur zwischen jeder Sonde und der jeweiligen Nukleinsäurezielsequenz größer als die Annealing-Temperatur zwischen den Sonden ist.

2. Verfahren zum Erhalten eines Satzes von Sonden zur Capture-Hybridisierung von Nukleinsäuren nach Anspruch 1, wobei das Verfahren durch ein Design-Tool durchgeführt wird und das Verfahren die folgenden Schritte umfasst:
Schritt a, Eingeben von Anfangssequenzinformationen und Design-Parametern und Ausgeben von Sondensequenzinformationen, wobei die Anfangssequenzinformationen Folgendes umfassen:
Gesamtsequenzinformationen, die Informationen von Sequenzen sind, die vor dem Erfassen in einer Bibliothek enthalten sein können; und
Zielsequenzinformationen, die Informationen über zu erfassende Sequenzen und Sequenzen sind, die zu vermeiden sind, die Sequenzen mit geringer Spezifität sind, wie etwa wiederholte Sequenzen in einer umfangreichen Sequenz;
wobei die Design-Parameter einen Annealing-Temperaturbereich und einen Sequenzlängenbereich zum Binden einer Sonde an eine Zielsequenz und einen Längenbereich einer Sondenbindungssequenz umfassen;
Schritt b, Ausschneiden aller Teilsequenzen mit einer Länge k, wobei k 20-80 nt ist, aus Vorwärtsstrangsequenzen und komplementären Strangsequenzen in der Gesamtsequenz und Zählen der Anzahl von Vorkommen jeder Teilsequenz und Erhalten einer durchschnittlichen Anzahl von Vorkommen;
Schritt c, Auswählen einer Sondenbindungssequenz, in der Sonden komplementär ein Paar bilden, wobei die Sondenbindungssequenz eine Länge k aufweist, wobei k 8-30 nt beträgt, und eine Annealing-Temperatur aufweist, die niedriger als die Annealing-Temperatur zum Binden der Sonde an die Zielsequenz ist, und das Vorkommen der Sondenbindungssequenz in der Gesamtsequenz weniger als 5 % der durchschnittlichen Anzahl von Vorkommen beträgt;
Schritt d, Auswählen von zielspezifischen Sequenzen, in denen jede Sonde an eine jeweilige Nukleinsäurezielsequenz bindet: zuerst wird eine i-te Zielsequenz ausgewählt, wobei i einen Anfangswert gleich 1 aufweist; und dann wird dann die jeweilige zielspezifische Sequenz ausgewählt, in der die Sonde an die i-te Zielsequenz bindet, ausgehend von einer n-ten Base der ausgewählten Zielsequenz, wobei n einen Anfangswert gleich 1 aufweist;
Schritt e, Hinzufügen der Sondenbindungssequenz an ein 5'-Ende jeder zielspezifischen Sequenz und Hinzufügen einer umgekehrten komplementären Sequenz der Sondenbindungssequenz an ein 3'-Ende jeder zielspezifischen Sequenz; und
Schritt f, Ausgeben aller Sondensequenzen.

3. Verwendung des Satzes von Sonden nach Anspruch 1 zum Nachweis von Niederfrequenzmutationen, zur Analyse von Chromosomenkopienzahlvariation, zur Insertion/Deletion, zur Mikrosatelliteninstabilität oder zur Fusionsgenvariation in DNA-Fragmenten.

4. Verwendung der Sonde nach Anspruch 1 zur gezielten metagenomischen Sequenzierung der nächsten Generation, mNGS, oder zum Nachweis von Pathogen-Epidemiologie.

## Revendications

1. Ensemble de sondes pour la capture par hybridation d'acides nucléiques, comprenant plusieurs sondes différentes, chaque sonde comprenant
un segment médian qui est une séquence spécifique cible respective s'appariant de manière complémentaire avec une séquence cible d'acide nucléique correspondante,
une première séquence de liaison à une sonde, qui est située au niveau de l'extrémité 5' de la sonde et s'apparie de manière complémentaire à une extrémité 3' d'une autre sonde,
et
une seconde séquence de liaison à une sonde, qui est située au niveau de l'extrémité 3' de la sonde et qui est appariée de manière complémentaire à une extrémité 5' d'une autre sonde ;
ladite première séquence de liaison à une sonde présentant une longueur de 8 à 30 nt, ladite seconde séquence de liaison à une sonde présentant une longueur de 8 à 30 nt,
ladite séquence spécifique cible respective présentant une longueur de 20 à 80 nt ;
ladite extrémité 3' ou ladite extrémité 5' de chaque sonde comportant un biomarqueur ;
ledit biomarqueur étant la biotine pour la liaison à la streptavidine sur des billes magnétiques ;
ladite température d'hybridation entre chaque sonde et la séquence cible d'acide nucléique respective étant supérieure à la température d'hybridation entre les sondes.

2. Procédé permettant l'obtention d'un ensemble de sondes pour la capture par hybridation d'acides nucléiques selon la revendication 1, ledit procédé étant réalisé par un outil de conception, et ledit procédé comprenant les étapes suivantes de :
étape a, entrée d'informations de séquence initiale et de paramètres de conception, et sortie d'informations de séquence de sonde, lesdites informations de séquence initiale comprenant
des informations de séquence totale, qui sont des informations de séquences qui peuvent être contenues avant la capture dans une bibliothèque ; et
des informations de séquence cible, qui sont des informations de séquences devant être capturées, et des séquences qui doivent être évitées, qui sont des séquences de faible spécificité telles que des séquences répétées dans une séquence complète ;
lesdits paramètres de conception comprenant une plage de température d'hybridation et une plage de longueur de séquence pour la liaison d'une sonde à une séquence cible, et une plage de longueur d'une séquence de liaison à une sonde ;
étape b, découpe de toutes les sous-séquences d'une longueur de k, où k est situé entre 20 et 80 nt, en partant des séquences de brins sens et des séquences de brins complémentaires dans la séquence totale, et le comptage du nombre d'occurrences de chaque sous-séquence, et l'obtention d'un nombre moyen d'occurrences ;
étape c, sélection d'une séquence de liaison à une sonde dans laquelle les sondes sont appariées de manière complémentaire, ladite séquence de liaison à une sonde présentant une longueur de k, où k est situé entre 8 et 30 nt, et présentant une température d'hybridation qui est inférieure à la température d'hybridation pour la liaison de la sonde à la séquence cible, et ladite occurrence de la séquence de liaison à une sonde dans la séquence totale étant inférieure à 5 % du nombre moyen d'occurrences ;
étape d, sélection des séquences spécifiques cibles dans lesquelles chaque sonde se lie à une séquence cible d'acide nucléique respective : tout d'abord, une i-ième séquence cible est sélectionnée, i présentant une valeur initiale égale à 1 ; et ensuite la séquence spécifique cible respective dans laquelle la sonde se lie à la i-ième séquence cible est ensuite sélectionnée, à partir d'une n-ième base de la séquence cible sélectionnée, n présentant une valeur initiale égale à 1 ;
étape e, ajout de la séquence de liaison à une sonde au niveau d'une extrémité 5' de chaque séquence spécifique cible, et ajout d'une séquence complémentaire inverse de la séquence de liaison à une sonde à une extrémité 3' de chaque séquence spécifique cible ; et
étape f, sortie de toutes les séquences de sonde.

3. Utilisation de l'ensemble de sondes selon la revendication 1 pour la détection de mutations à basse fréquence, l'analyse de la variation du nombre de copies chromosomiques, l'insertion/délétion, l'instabilité des microsatellites ou la variation du gène de fusion dans des fragments d'ADN.

4. Utilisation de la sonde selon la revendication 1 pour le séquençage métagénomique ciblé de nouvelle génération, mNGS, ou la détection de l'épidémiologie des agents pathogènes.
